# EUROPEAN PATENT APPLICATION

(11) **EP 4 345 109 A1**
(43) Date of publication of application: **03.04.2024**
(21) Application number: 22199273.8
(22) Date of filing: 30.09.2022
(51) Int. Cl.: C07K 16/22, A61K 39/395, A61P 9/14, G01N 33/53

(54) **ANTI-ADRENOMEDULLIN (ADM) BINDER FOR USE IN THERAPY OF PEDIATRIC PATIENTS WITH CONGENITAL HEART DISEASE**

(71) Applicant: AdrenoMed AG, 16761 Hennigsdorf (DE)
(72) Inventor: The designation of the inventor has not yet been filed
(74) Representative: Kilger, Ute

(57) **Abstract**

Subject matter of the present invention is an anti-adrenomedullin (ADM) antibody or an anti-adrenomedullin antibody fragment or anti-ADM non-Ig scaffold for the prevention or treatment of a pleural effusion in a pediatric patient with congenital heart disease that is undergoing or underwent cardiac surgery with cardio-pulmonary bypass, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of pro-Adrenomedullin (pro-ADM) or a fragment thereof above a predetermined threshold in a sample of bodily fluid of said patient, wherein said pro-ADM or fragment is selected from the group comprising pro-ADM according to SEQ ID No. 1 or PAMP according to SEQ ID No.: 3 or MR-proADM according to SEQ ID No.: 4 or mature ADM-NH₂ (bio-ADM) according to SEQ ID No.: 5 or ADM-Gly according to SEQ ID No.: 6 or CT-proADM according to SEQ ID No.: 7.

## Description

Subject matter of the present invention is an anti-adrenomedullin (ADM) antibody or an anti-adrenomedullin antibody fragment or anti-ADM non-Ig scaffold for the prevention or treatment of a pleural effusion in a pediatric patient with congenital heart disease that is undergoing or underwent cardiac surgery with cardio-pulmonary bypass, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of pro-Adrenomedullin (pro-ADM) or a fragment thereof above a predetermined threshold in a sample of bodily fluid of said patient, wherein said pro-ADM or fragment is selected from the group comprising pro-ADM according to SEQ ID No. 1 or PAMP according to SEQ ID No.: 3 or MR-proADM according to SEQ ID No.: 4 or mature ADM-NH₂ (bio-ADM) according to SEQ ID No.: 5 or ADM-Gly according to SEQ ID No.: 6 or CT-proADM according to SEQ ID No.: 7.

### Background

About 1 in 1000 live births is born with a structural heart defect. Complex cardiac vitia (e.g. tetralogy of Fallot, transposition of the large vessels, hypoplastic left heart syndrome, Ebstein anomaly, etc.) have a lower prevalence and are therefore classified as "rare diseases" / "orphan diseases".

Most patients undergo an intervention (cardiac catheter) or an operation in newborns or early childhood.

In children with congenital heart defects who are undergoing heart surgery, the Cardiopulmonary Bypass (CPB) pump is required intraoperatively, and in some cases the extracorporeal membrane oxygenation (ECMO) is required postoperatively in the pediatric intensive care unit. ECMO is also used in acute heart failure in pediatric intensive care medicine, regardless of surgery, in children with structural heart disease.

Capillary leak from CPB pump and ECMO is associated with morbidity and mortality.

The extracorporeal circulation is used in children with congenital heart defects during an operation on the heart (CPB), or in the pediatric intensive care unit for heart failure (ECMO). In the case of extracorporeal circulation, the contact of blood with foreign membranes can lead to a systemic inflammatory reaction (SIR) (Day, J.R. and Taylor, K.M. (2005) Int J Surg 3; 129-40), (Elgebaly, S.A., et al. (1994) Ann Thorac Surg 57; 391-6), (Cremer, J., et al. (1996) Ann Thorac Surg 61; 1714-20), (Kirklin, J.K., et al. (1987) Blood Purif 5; 168-78), (Levy, J.H. and Kelly, A.B. (1993) Can J Anaesth 40; 1009-15), (Prasad, K., et al. (1992) American heart journal 123; 37-45). This can cause increased permeability of the capillaries, which results in the leakage of plasma and plasma proteins into the interstitium (Seghaye, M.C., et al. (1996) J Thorac Cardiovasc Surg 112; 687-97). This leads to mild to severe complications, such as pleural or pericardial effusions, ascites, edema, acute lung failure or arterial hypotension (Tarnok, A., et al. (1999) Pediatr Cardiol 20; 113-25). The extreme case is the "capillary leak syndrome" with the consequence of multiple organ failure (Teelucksingh, S., et al. (1990) Q J Med 75; 515-24).

The early detection of a systemic inflammatory reaction and a capillary leak syndrome by means of appropriate biomarkers can considerably reduce the morbidity and mortality of children with congenital heart defects in extracorporeal circulation through timely therapeutic measures (Seghaye, M.C. (2003) Cardiol Young 13; 228-39), (Kubicki, R., et al. (2013) Eur J Cardiothorac Surg 44; 275-81).

### Adrenomedullin in children with congenital heart disease

Plasma levels of Adrenomedullin in children with congenital heart disease have been investigated earlier: It has been described that plasma ADM levels are increased in congenital heart disease with high pulmonary blood flow and hypertension or with cyanosis and that they are related to pulmonary arterial resistance and hypoxemia (Zhu, X.B., et al. (2006) Zhongguo Dang Dai Er Ke Za Zhi 8; 90-2).

Low cardiac output syndrome (LCOS) remains a major perioperative complication in children after congenital heart disease surgery involving CPB. A correlation of plasma ADM measured at the end of CPB with LCOS was found (Abella, R., et al. (2012) J Matern Fetal Neonatal Med 25; 2756-61). Several studies have demonstrated that MR-proADM, a non-functional circulating peptide derived from the same precursor as ADM, alone or combined with other parameters may be a predictor of low cardiac output syndrome (LCOS) in children undergoing CPB (Wu, A.H. and Hale, K. (2015) Critical care medicine 43; 494-5, Takeuchi, M., et al. (2001) Acta Med Okayama 55; 245-52). A cut-off point of 1.5 nmol/L for MR-proADM at 2 and 24 h post-CPB, respectively, has been shown to allow determination of LCOS with high sensitivity and specificity (Perez-Navero, J.L., et al. (2017) Rev Esp Cardiol (Engl Ed) 70; 267-74). Pre-operative elevation of MR-proADM has been described to predict increased respiratory and inotropic support after cardiac surgery as well as other complications including neurological disability, renal replacement therapy, prolonged length of stay (Bobillo-Perez, S., et al. (2020) PloS one 15; e0236377). In another study in newborns and infants under 2 months admitted to an intensive care unit after cardiac surgery, elevated MR-proADM determined via the first blood test performed in the ICU after cardiac surgery predicted poor outcome (defined as mortality, cardiac arrest, requiring extracorporeal support, requiring renal replacement therapy, or neurological injury) with an area under the curve of 0.735, and mortality alone with an area under the curve of 0.869 (Bobillo-Perez, S., et al. (2021) Eur J Pediatr).

There is an ongoing need for new forms of therapy or prevention of pleural effusion in a pediatric patient with congenital heart disease that is undergoing or underwent cardiac surgery with cardio-pulmonary bypass. The present invention addresses this need.

The peptide adrenomedullin (ADM) was described for the first time in 1993 (Kitamura K. et al. 1993. Biochemical and Biophysical Research Communications Vol. 192 (2): 553-560*)* as a novel hypotensive peptide comprising 52 amino acids, which had been isolated from a human pheochromocytome. In the same year, cDNA coding for a precursor peptide comprising 185 amino acids and the complete amino acid sequence of this precursor peptide were also described. The precursor peptide, which comprises, inter alia, a signal sequence of 21 amino acids at the N-terminus, is referred to as "preproadrenomedullin" (pre-proADM). In the present description, all amino acid positions specified usually relate to the pre-proADM which comprises the 185 amino acids. The peptide adrenomedullin (ADM) is a peptide which comprises 52 amino acids (SEQ ID No: 1) and which comprises the amino acids 95 to 146 of pre-proADM, from which it is formed by proteolytic cleavage. To date, only a few fragments of the peptide fragments formed in the cleavage of the pre-proADM have been more exactly characterized, in particular the physiologically active peptides adrenomedullin (ADM) and "PAMP", a peptide comprising 20 amino acids (22-41) which follows the 21 amino acids of the signal peptide in pre-proADM. The discovery and characterization of ADM in 1993 triggered intensive research activity, the results of which have been summarized in various review articles, in the context of the present description, reference being made in particular to the articles to be found in an issue of "Peptides" devoted to ADM in particular (*Editorial*, Takahashi K. 2001. Peptides 22:1691*) and (*Eto T. 2001. Peptides 22: 1693-1711). A further review is (Hinson et al. 2000. Endocrine Reviews 21(2):138-167)*.* In the scientific investigations to date, it has been found, inter alia, that ADM may be regarded as a poly-functional regulatory peptide. It is released into the circulation in an inactive form extended by glycine (Kitamura K. et al. 1998. Biochem. Biophys. Res. Commun. 244(2):551-555)*.* There is also a binding protein (Pio R. et al. 2001. The Journal of Biological Chemistry 2 76(15):12292-12300) which is specific for ADM and probably likewise modulates the effect of ADM. Those physiological effects of ADM as well as of PAMP, which are of primary importance in the investigations to date, were the effects influencing blood pressure.

ADM is an effective vasodilator, and it is possible to associate the hypotensive effect with the particular peptide segments in the C-terminal part of ADM. It has furthermore been found that the above-mentioned further physiologically active peptide PAMP formed from pre-proADM likewise exhibits a hypotensive effect, even if it appears to have an action mechanism differing from that of ADM.

It has furthermore been found that the concentrations of ADM, which can be measured in the circulation and other biological liquids are, in a number of pathological states, significantly above the concentrations to be found in healthy control persons. Thus, the ADM level in patients with congestive heart failure, myocardial infarction, kidney diseases, hypertensive disorders, diabetes mellitus, in the acute phase of shock and in sepsis and septic shock are significantly increased, although to different extents. The PAMP concentrations are also increased in some of said pathological states, but the plasma levels are lower relative to ADM ((Eto, T., supra). It is furthermore known that unusually high concentrations of ADM are to be observed in sepsis, and the highest concentrations in septic shock (cf. (Eto, T., "supra) and (Hirata et al. Journal of Clinical Endocrinology and Metabolism 1996. 81(4): 1449-1453*;* Ehlenz K. et al. 1997. Exp Clin Endocrinol Diabetes 105: 156-162 *);* Tomoda Y. et al. 2001. Peptides 22: 1783-1794 *;* Ueda S. et al. 1999. Am. J. Respir. Crit. Care Med. 160: 132-136*: and* Wang P. Peptides 2001. 22: 1835-1840).

### Definitions

Before describing the invention in detail, it is deemed expedient to provide definitions for certain technical terms used throughout the description. Although the present invention will be described with respect to particular embodiments, this description is not to be construed in a limiting sense. Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting" of is considered to be a preferred embodiment of the term "comprising" of. If hereinafter a group is defined to comprise at least a certain number of embodiments, this is meant to also encompass a group, which preferably consists of these embodiments only.

It is also to be understood that the terminology used herein is for the purpose of describing particular embodiments only, and is not intended to limit the scope of the present invention that will be limited only by the appended claims.

As used herein, the term "pleural effusion" refers to an unusual amount of fluid around the lung. Pleural effusion, sometimes referred to as "water on the lungs," is the build-up of excess fluid between the layers of the pleura outside the lungs. The pleura are thin membranes that line the lungs and the inside of the chest cavity and act to lubricate and facilitate breathing. Normally, a small amount of fluid is present in the pleura.

As used herein, the term "congenital heart disease" is defined as a range of birth defects that affect the normal way the heart works; the term "congenital" means the condition is present from birth.

The term "elevated level" means a level above a certain threshold level.

A bodily fluid may be selected from the group comprising blood, serum, plasma, urine, cerebrospinal liquid (CSF), and saliva.

As used herein, an "effective dosage" or "effective amount" of drug, compound, antibody, antibody fragment or pharmaceutical composition is an amount sufficient to effect beneficial or desired results. For prophylactic use, beneficial or desired results include results such as eliminating or reducing the risk, lessening the severity, or delaying the outset of the disease, including biochemical, histological and/or behavioral symptoms of the disease, its complications and intermediate pathological phenotypes presenting during development of the disease. For therapeutic use, beneficial or desired results include clinical results such as reducing pain intensity, duration, or frequency of headache attack, and decreasing one or more symptoms resulting from headache (biochemical, histological and/or behavioral), including its complications and intermediate pathological phenotypes presenting during development of the disease, increasing the quality of life of those suffering from the disease, decreasing the dose of other medications required to treat the disease, enhancing effect of another medication, and/or delaying the progression of the disease of patients. An effective dosage can be administered in one or more administrations. For purposes of this invention, an effective dosage of drug, compound, or pharmaceutical composition is an amount sufficient to accomplish prophylactic or therapeutic treatment either directly or indirectly. As is understood in the clinical context, an effective dosage of a drug, compound, or pharmaceutical composition may or may not be achieved in conjunction with another drug, compound, or pharmaceutical composition. Thus, an "effective dosage" may be considered in the context of administering one or more therapeutic agents, and a single agent may be considered to be given in an effective amount if, in conjunction with one or more other agents, a desirable result may be or is achieved.

Mature ADM, bio-ADM and ADM-NH₂ is used synonymously throughout this application and is a molecule according to SEQ ID No.: 5.

### Detailed description of embodiments of the invention

Below, embodiments of the invention are provided. It is noted that generally embodiments can be combined with any other embodiment of the same category (product, process, use, method).

Subject-matter of the present invention is an anti-adrenomedullin (ADM) antibody or antibody fragment for the prevention or treatment of a pleural effusion in a pediatric patient with congenital heart disease that is undergoing or underwent cardiac surgery with cardio-pulmonary bypass, wherein said antibody or antibody fragment binds to the N-terminal part (aa 1-21) of ADM having the SEQ ID NO. 2 (YRQSMNNFQGLRSFGCRFGTC) and wherein said antibody or antibody fragment is administered to a patient having a level of pro-Adrenomedullin or a fragment thereof above a predetermined threshold in a sample of bodily fluid of said patient, wherein said Pro-Adrenomedullin or fragment is selected from the group comprising Pro-Adrenomedullin according to SEQ ID No. 1 or PAMP according to SEQ ID No.: 3 or MR-proADM according to SEQ ID No.: 4 or mature ADM-NH₂ (bio-ADM) according to SEQ ID No.: 5 or ADM-Gly according to SEQ ID No.: 6 or CT-proADM according to SEQ ID No.: 7.

One embodiment of the present application relates to an anti-adrenomedullin (ADM) antibody or antibody fragment for the prevention or treatment of a pleural effusion in a pediatric patient with congenital heart disease that is undergoing or underwent cardiac surgery with cardio-pulmonary bypass, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of pro-Adrenomedullin or fragments thereof above a predetermined threshold in a sample of bodily fluid of said patient.

One embodiment of the present application relates to an anti-adrenomedullin (ADM) antibody or antibody fragment for the prevention or treatment of a pleural effusion in a pediatric patient with congenital heart disease that is undergoing or underwent cardiac surgery with cardio-pulmonary bypass, and wherein said antibody or antibody fragment is administered to a patient with a level of pro-Adrenomedullin or a fragment thereof above a predetermined threshold, which is the 95th percentile of the normal range of pro-Adrenomedullin or a fragment thereof of a healthy subject, more preferred which is the 96th percentile of the normal range of bio-ADM of a healthy subject, even more preferred which is the 97th percentile of the normal range of bio-ADM of a healthy subject, wherein said sample of bodily fluid is selected from the group comprising whole blood, plasma, and serum, in particular blood plasma.

Another embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, and wherein said antibody or antibody fragment is administered to a patient with a level of pro-Adrenomedullin or a fragment thereof above a predetermined threshold, which is for the amount of bio-ADM in a sample of bodily fluid ≥ 43 pg/ml, wherein said sample of bodily fluid is selected from the group comprising whole blood, plasma, and serum, in particular blood plasma.

In a specific embodiment of the invention the predetermined threshold of MR-proADM in a sample of bodily fluid of said subject is within a threshold range that is between 0.5 and 1.5 nmol/L, preferably between 0.7 and 1 nmol/L, most preferred a threshold of 0.8 nmol/L is applied.

In a specific embodiment of the invention the predetermined threshold of bio-ADM in a sample of bodily fluid of said subject is between 40 and 100 pg/mL, more preferred between 50 and 90 pg/mL, even more preferred between 60 and 80 pg/mL, most preferred said threshold is 70 pg/mL.

In a specific embodiment of the invention the predetermined threshold of CT-proADM in a sample of bodily fluid of said subject is within a threshold range that is between 85 and 350 pmol/L, preferably between 100 and 250 pmol/L, most preferred a threshold of 150 pmol/L is applied.

In a specific embodiment of the invention the predetermined threshold of PAMP-amide in a sample of bodily fluid of said subject is within a threshold range that is between 0.3 and 1.2 pmol/L, preferably between 0.4 and 1.0 pmol/L, most preferred a threshold of 0.8 pmol/L is applied.

In a specific embodiment of the invention the predetermined threshold of PAMP-glycine in a sample of bodily fluid of said subject is within a threshold range that is between 0.5 and 2.0 pmol/L, preferably between 0.7 and 1.8 pmol/L, most preferred a threshold of 1.5 pmol/L is applied.

In a specific embodiment of the invention a threshold for bio-ADM in a sample of bodily fluid of said subject is the 5fold median concentration, preferably the 4fold median concentration, more preferred the 3fold median concentration, most preferred the 2fold median concentration of a normal healthy population.

In a specific embodiment of the invention a threshold for MR-proADM in a sample of bodily fluid of said subject is the 5fold median concentration, preferably the 4fold median concentration, more preferred the 3fold median concentration, most preferred the 2fold median concentration of a normal healthy population.

In a specific embodiment of the invention a threshold for CT-proADM in a sample of bodily fluid of said subject is the 5fold median concentration, preferably the 4fold median concentration, more preferred the 3fold median concentration, most preferred the 2fold median concentration of a normal healthy population.

In a specific embodiment of the invention a threshold for ADM-Gly in a sample of bodily fluid of said subject is the 5fold median concentration, preferably the 4fold median concentration, more preferred the 3fold median concentration, most preferred the 2fold median concentration of a normal healthy population.

In a specific embodiment of the invention a threshold for PAMP in a sample of bodily fluid of said subject is the 5fold median concentration, preferably the 4fold median concentration, more preferred the 3fold median concentration, most preferred the 2fold median concentration of a normal healthy population.

As used herein, the term "PAMP" comprises both circulating forms of PAMP, namely a biologically inactive C-terminally Glycine-extended PAMP (PAMP-Gly) and a biologically active C-terminally amidated PAMP (PAMP-amide).

Threshold levels can be obtained for instance from a Kaplan-Meier analysis, where the occurrence of a disease is correlated with the quartiles of the biomarker in the population. According to this analysis, subjects with biomarker levels above the 75th percentile have a significantly increased risk for getting the diseases according to the invention. This result is further supported by Cox regression analysis with full adjustment for classical risk factors: The highest quartile versus all other subjects is highly significantly associated with increased risk for getting a disease according to the invention.

Other preferred cut-off values are for instance the 90th, 95th or 99th percentile of a normal population. By using a higher percentile than the 75th percentile, one reduces the number of false positive subjects identified, but one might miss to identify subjects, who are at moderate, albeit still increased risk. Thus, one might adopt the cut-off value depending on whether it is considered more appropriate to identify most of the subjects at risk at the expense of also identifying "false positives", or whether it is considered more appropriate to identify mainly the subjects at high risk at the expense of missing several subjects at moderate risk.

The above-mentioned threshold values might be different in other assays, if these have been calibrated differently from the assay system used in the present invention. Therefore, the above-mentioned threshold shall apply for such differently calibrated assays, accordingly, taking into account the differences in calibration. One possibility of quantifying the difference in calibration is a method comparison analysis (correlation) of the assay in question (e.g., bio-ADM assay) with the respective biomarker assay used in the present invention by measuring the respective biomarker (e.g., bio-ADM) in samples using both methods. Another possibility is to determine with the assay in question, given this test has sufficient analytical sensitivity, the median biomarker level of a representative normal population, compare results with the median biomarker levels as described in the literature (*e.g.,* Weber et al. 2017. JALM2(2): 222-233) and recalculate the calibration based on the difference obtained by this comparison. With the calibration used in the present invention, samples from normal (healthy) subjects have been measured: median plasma bio-ADM (mature ADM-NH₂) was 13.7 pg/ml (inter quartile range [IQR] 9.6 - 18.7 pg/mL) *(*Weber et al. 2017. JALM2(2): 222-233).

Other methods to quantify fragments derived from the ADM precursor have been described, e.g., the measurement of MR-proADM (Morgenthaler et al. 2005. Clin Chem 51(10):1823-9), PAMP (Washimine et al. 1994. Biochem Biophys Res Commun 202(2):1081-7) and CT-proADM (EP 2 111 552). A commercial homogeneous time-resolved fluoroimmunoassay for the measurement of MR-proADM in plasma on a fully automated system is available (BRAHMS MR-proADM KRYPTOR; BRAHMS GmbH, Hennigsdorf, Germany) (Caruhel et al. 2009. Clin Biochem 42(7-8): 725-8). As these peptides are generated in a stoichiometric ratio from the same precursor, their plasma levels are correlated to a certain extent.

Another preferred embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, and wherein said antibody or antibody fragment is administered to a patient having a level of bio-ADM, wherein such level is determined from a sample of bodily fluid taken pre-, intra- or post operatively.

Another embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, and wherein said antibody or antibody fragment is administered to a patient having a level of pro-Adrenomedullin or fragments thereof, wherein the level of pro-Adrenomedullin or fragments thereof is determined in a bodily fluid obtained from said patient with an immunoassay.

Another specific embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, wherein said antibody or antibody fragment may be administered in a dose of at least 0.5 mg /kg body weight, particularly at least 1.0 mg/kg body weight, more particularly, from 1.0 to 20.0 mg/kg body weight, e.g., from 2.0 to 10 mg/kg body weight, from 2.0 to 8.0 mg/kg body weight, or from 2.0 to 5.0 mg/kg body weight.

Another specific embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, wherein said antibody or antibody fragment may be administered to the patient only once.

Another specific embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, wherein said antibody or antibody fragment may be administered to the patient several times with a time interval of at least one hour.

Another embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, wherein said antibody or antibody fragment is a human monoclonal antibody or a fragment that binds to the N-terminal region (aa 1-21) of ADM (SEQ ID No. 2) or an antibody fragment thereof wherein the heavy chain comprises the sequences:
CDR1: SEQ ID No: 8
   GYTFSRYW
CDR2: SEQ ID No: 9
   ILPGSGST
CDR3: SEQ ID No: 10
   TEGYEYDGFDY
and wherein the light chain comprises the sequences:
CDR1: SEQ ID No: 11
   QSIVYSNGNTY
CDR2:
   RVS
CDR3: SEQ ID No: 12
   FQGSHIPYT.

Another embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, wherein said antibody or fragment comprises the following sequence as a heavy chain: 
SEQ ID NO: 22

QVQLVQSGAEVKKPGSSVKVSCKASGYTFSRYWIEWVRQAPGQGLEWIGEILPGSGS
TNYNQKFQGRVTITADTSTSTAYMELSSLRSEDTAVYYCTEGYEYDGFDYWGQGTT
VTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPP
 
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
or a sequence that is > 95% identical to it, preferably > 98%, preferably > 99% and comprises the following sequence as a light chain: 
SEQ ID NO: 23

DVVLTQSPLSLPVTLGQPASISCRSSQSIVYSNGNTYLEWYLQRPGQSPRLLIYRVSNR
FSGVPDRFSGSGSGTDFTLKISRVEAEDVGVYYCFQGSHIPYTFGGGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
or a sequence that is > 95% identical to it, preferably > 98%, preferably > 99%.

To assess the identity between two amino acid sequences, a pairwise alignment is performed. Identity defines the percentage of amino acids with a direct match in the alignment.

Another embodiment of the present application relates to an anti-ADM antibody or antibody fragment for the prevention or treatment of a pleural effusion in a pediatric patient with congenital heart disease that is undergoing or underwent cardiac surgery with cardio-pulmonary bypass, wherein said antibody or antibody fragments are administered orally, intravenously, subcutaneously, intraarterially, intramuscularly, intracardially, intraspinally, intrathoracically, intraperitoneal, intraventricular, sublingually, transdermal, and/or via inhalation.

Furthermore, in embodiments of the invention an anti-ADM antibody or an anti-ADM antibody fragment is monospecific. Monospecific anti-ADM antibody or monospecific anti-ADM antibody fragment means that said antibody or antibody fragment binds to one specific region encompassing at least 5 amino acids within the target ADM. Monospecific anti-ADM antibody or monospecific anti-ADM antibody fragment are anti-ADM antibodies or anti-ADM antibody fragments that all have affinity for the same antigen.

In a specific and preferred embodiment the present invention provides for a monospecific anti-ADM antibody or monospecific anti-ADM antibody fragment, characterized in that said antibody or antibody fragment binds to one specific region encompassing at least 4 amino acids within the target ADM. In another special embodiment the anti-ADM antibody or the antibody fragment binding to ADM is a monospecific antibody. Monospecific means that said antibody or antibody fragment binds to one specific region encompassing preferably at least 4, or at least 5 amino acids within the target ADM. Monospecific antibodies or fragments are antibodies or fragments that all have affinity for the same antigen. Monoclonal antibodies are monospecific, but monospecific antibodies may also be produced by other means than producing them from a common germ cell.

An antibody according to the present invention is a protein including one or more polypeptides substantially encoded by immunoglobulin genes that specifically binds an antigen. The recognized immunoglobulin genes include the kappa, lambda, alpha (IgA), gamma (IgG1, IgG2, IgG3, IgG4), delta (IgD), epsilon (IgE) and mu (IgM) constant region genes, as well as the myriad immunoglobulin variable region genes. Full-length immunoglobulin light chains are generally about 25 kDa or 214 amino acids in length. Full- length immunoglobulin heavy chains are generally about 50 kDa or 446 amino acids in length. Light chains are encoded by a variable region gene at the NH₂-terminus (about 110 amino acids in length) and a kappa or lambda constant region gene at the COOH--terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')2, as well as bifunctional hybrid antibodies and single chains (e.g., Lanzavecchia et al. 1987. Eur. J. Immunol. 17: 105*:* Huston et al 1988. PNAS 85:5879-5883*;* Bird et al. 1988. Science 242:423-426*;* Hood et al. 1984. Immunology. Benjamin. N.Y., 2nd ed*.;* Hunkapiller and Hood, 1986. Nature 323: 15-16). An immunoglobulin light or heavy chain variable region includes a framework region interrupted by three hypervariable regions, also called complementarity determining regions (CDR's) (see, Sequences of Proteins of Immunological Interest, E. Kabat et al, U.S. Department of Health and Human Services, 1983).

As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art, e.g., see U.S. Patent No. 5,807,715. A "humanized" immunoglobulin is an immunoglobulin including a human framework region and one or more CDRs from a non-human (such as a mouse, rat, or synthetic) immunoglobulin. The non- human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, i.e., at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions, which have substantially no effect on antigen binding or other immunoglobulin functions. Exemplary conservative substitutions are those such as gly, ala; val, ile, leu; asp, glu; asn, gin; ser, thr; lys, arg; and phe, tyr. Humanized immunoglobulins can be constructed by means of genetic engineering (e.g., see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, e.g., Dower et al., PCT Publication No. WO 91/17271; McCafferty et al.; PCT Publication No. WO92/001047; and Winter, PCT Publication No. WO 92/20791), or selected from a human combinatorial monoclonal antibody library (see the Morphosys website). Human antibodies can also be prepared by using transgenic animals carrying a human immunoglobulin gene (for example, see PCT Publication No. WO 93/12227; and PCT Publication No. WO 91/10741).

Thus, the anti-ADM antibody may have the formats known in the art. Examples are human antibodies, monoclonal antibodies, humanized antibodies, chimeric antibodies, CDR-grafted antibodies. In a preferred embodiment antibodies according to the present invention are recombinantly produced antibodies as e.g. IgG, a typical full-length immunoglobulin, or antibody fragments containing at least the F-variable domain of heavy and/or light chain as e.g. chemically coupled antibodies (fragment antigen binding) including but not limited to Fab-fragments including Fab minibodies, single chain Fab antibody, monovalent Fab antibody with epitope tags, e.g. Fab-V5Sx2; bivalent Fab (mini-antibody) dimerized with the CH3 domain; bivalent Fab or multivalent Fab, e.g. formed via multirnerization with the aid of a heterologous domain, e.g. via dimerization of dHLX domains,e.g. Fab-dHLX-FSx2; F(ab')2-fragments, scFv-fragments, multimerized multivalent or/and multispecific scFv- fragments, bivalent and/or bispecific diabodies, BITE^{®} (bispecific T-cell engager), trifunctional antibodies, polyvalent antibodies, e.g. from a different class than G; single-domain antibodies, e.g. nanobodies derived from camelid or fish immunoglobulins and numerous others.

In addition to anti-ADM antibodies other biopolymer scaffolds are well known in the art to complex a target molecule and have been used for the generation of highly target specific biopolymers. Examples are aptamers, spiegelmers, anticalins and conotoxins. For illustration of antibody formats please see Fig. 1 a, 1 b and 1 c in WO 2013/072513.

An antibody fragment according to the present invention is an antigen binding fragment of an antibody according to the present invention.

In a preferred embodiment the ADM antibody format is selected from the group comprising Fv fragment, scFv fragment, Fab fragment, scFab fragment, (Fab)2 fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is the scFab format.

In one embodiment of the invention antibodies according to the present invention may be produced as follows: A Balb/c mouse was immunized with 100ug ADM-Peptide-BSA-Conjugate at day 0 and 14 (emulsified in 100µI complete Freund's adjuvant) and 50µg at day 21 and 28 (in 100 µl incomplete Freund's adjuvant). Three days before the fusion experiment was performed, the animal received 50µg of the conjugate dissolved in 100 µI saline, given as one intraperitoneal and one intravenous injection. Splenocytes from the immunized mouse and cells of the myeloma cell line SP2/0 were fused with 1ml 50% polyethylene glycol for 30s at 37°C. After washing, the cells were seeded in 96-well cell culture plates. Hybrid clones were selected by growing in HAT medium (RPMI 1640 culture medium supplemented with 20% fetal calf serum and HAT-Supplement). After two weeks the HAT medium is replaced with HT Medium for three passages followed by returning to the normal cell culture medium. The cell culture supernatants were primary screened for antigen specific IgG antibodies three weeks after fusion. The positive tested microcultures were transferred into 24-well plates for propagation. After retesting, the selected cultures were cloned and re-cloned using the limiting-dilution technique and the isotypes were determined (see also Lane. R.D. 1985. J. Immunol. Meth. 81: 223-228*;* Ziegler B. et al. 1996. Horm. Metab. Res. 28: 11-15).

Antibodies may also be produced by means of phage display according to the following procedure: The human naive antibody gene libraries HAL7/8 were used for the isolation of recombinant single chain F- Variable domains (scFv) against ADM peptide. The antibody gene libraries were screened with a panning strategy comprising the use of peptides containing a biotin tag linked via two different spacers to the ADM peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatants from the cultivation of these clonal strains have been directly used for an antigen ELISA testing (see references cited in WO 2013/072513, incorporated herein in their entirety).

Humanization of murine antibodies may be conducted according to the following procedure: For humanization of an antibody of murine origin the antibody sequence is analyzed for the structural interaction of framework regions (FR) with the complementary determining regions (CDR) and the antigen. Based on structural modelling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modeling (Almagro and Fransson 2008. Front Biosci. 13: 1619-33) in a preferred embodiment the ADM antibody format is selected from the group comprising Fv fragment, scFv fragment, Fab fragment, scFab fragment, F(ab)2 fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is scFab format. In another preferred embodiment, the anti-ADM antibody, anti-ADM antibody fragment, or anti-ADM non-Ig scaffold is a full-length antibody, antibody fragment, or non-Ig scaffold.

In a preferred embodiment the anti-ADM antibody or an anti-ADM antibody fragment is directed to and can bind to an epitope of at least 5 amino acids in length contained in ADM.

In another preferred embodiment the anti-ADM antibody or an anti-ADM antibody fragment is directed to and can bind to an epitope of at least 4 amino acids in length contained in ADM.

In one specific embodiment of the invention the anti-ADM antibody or anti-ADM antibody fragment binding to ADM is provided for use for the prevention or treatment of a pleural effusion in a pediatric patient with congenital heart disease that is undergoing or underwent cardiac surgery with cardio-pulmonary bypass, wherein said antibody or fragment or scaffold is not ADM-binding- Protein-1 (complement factor H).

In another specific embodiment of the invention the anti-ADM antibody or anti-ADM antibody fragment binding to ADM is provided for use for the prevention or treatment of a pleural effusion in a pediatric patient with congenital heart disease that is undergoing or underwent cardiac surgery with cardio-pulmonary bypass, wherein said antibody or antibody fragment or non-Ig scaffold binds to a region of preferably at least 4, or at least 5 amino acids within the sequence of aa 1-42 of mature human ADM.

In one specific embodiment of the invention the anti-ADM antibody or anti-ADM antibody fragment binding to ADM is provided for use for the prevention or treatment of a pleural effusion in a pediatric patient with congenital heart disease that is undergoing or underwent cardiac surgery with cardio-pulmonary bypass, wherein said antibody or fragment or scaffold binds to a region of preferably at least 4, or at least 5 amino acids within the sequence of aa 1-21 of mature human ADM:
YRQSMNNFQGLRSFGCRFGTC (SEQ ID NO.: 2).

In a preferred embodiment of the present invention said anti-ADM antibody or an anti-ADM antibody fragment binds to a region of ADM that is located in the N-terminal part (amino acids 1-21) of ADM.

In another preferred embodiment said anti-ADM antibody or an anti-ADM antibody fragment recognizes and binds to the N-terminal end (aa1) of ADM. N-terminal end means that the amino acid 1, that is "Y" of SEQ ID No. 1 or 2 is mandatory for antibody binding. Said antibody or fragment would neither bind N-terminal extended nor N-terminally modified ADM nor N-terminally degraded ADM.

In a preferred embodiment the anti-ADM antibody or an anti-ADM antibody fragment is directed to and can bind to an epitope of at least 5 amino acids in length contained in ADM, preferably in human ADM.

In a preferred embodiment the anti-ADM antibody or an anti-ADM antibody fragment is directed to and can bind to an epitope of at least 4 amino acids in length contained in ADM, preferably in human ADM.

In one specific embodiment it is preferred to use an anti-ADM antibody or an anti-ADM antibody fragment according to the present invention, wherein said anti-ADM antibody or said anti-ADM antibody fragment is an ADM stabilizing antibody or an ADM stabilizing antibody fragment that enhances the half-life (t_{1/2}; half retention time) of ADM in serum, blood, plasma at least 10 %, preferably at least 50 %, more preferably >50 %, most preferably >100%. The half-life (half retention time) of ADM may be determined in human plasma in absence and presence of an ADM stabilizing antibody or an ADM stabilizing antibody fragment, respectively, using an immunoassay for the quantification of ADM.

The following steps may be conducted:
- ADM may be diluted in human citrate plasma in absence and presence of an ADM stabilizing antibody or an ADM stabilizing antibody fragment, respectively, and may be incubated at 24 °C;
- Aliquots are taken at selected time points (e.g. within 24 hours) and degradation of ADM may be stopped in said aliquots by freezing at -20 °C;
- The quantity of ADM may be determined by a hADM immunoassay directly, if the selected assay is not influenced by the stabilizing antibody. Alternatively, the aliquot may be treated with denaturing agents (like HCI) and, after clearing the sample (e.g. by centrifugation) the pH can be neutralized and the ADM-quantified by an ADM immunoassay. Alternatively, non-immunoassay technologies (e.g., RP-HPLC) can be used for ADM-quantification.
- The half-life of ADM is calculated for ADM incubated in absence and presence of an ADM stabilizing antibody or an ADM stabilizing antibody fragment, respectively.
- The enhancement of half-life is calculated for the stabilized ADM in comparison to ADM that has been incubated in absence of an ADM stabilizing antibody or an ADM stabilizing antibody fragment.

A two-fold increase of the half-life of ADM is an enhancement of half-life of 100%. Half Life (half retention time) is defined as the period over which the concentration of a specified chemical or drug takes to fall to half baseline concentration in the specified fluid or blood.

In a specific embodiment said anti-ADM antibody, anti-ADM antibody fragment is a non-neutralizing antibody or fragment. A neutralizing anti-ADM antibody or anti-ADM antibody fragment would block the bioactivity of ADM to nearly 100%, to at least more than 90%, preferably to at least more than 95%.

In contrast, a non-neutralizing anti-ADM antibody or anti-ADM antibody fragment blocks the bioactivity of ADM less than 100%, preferably to less than 95%, preferably to less than 90%, more preferred to less than 80 % and even more preferred to less than 50 %. This means that the residual bioactivity of ADM bound to the non-neutralizing anti-ADM antibody or anti-ADM antibody fragment would be more than 0%, preferably more than 5 %, preferably more than 10 %, more preferred more than 20 %, more preferred more than 50 %. In this context (a) molecule(s), being it an antibody, or an antibody fragment or a non-Ig scaffold with "non-neutralizing anti-ADM activity", collectively termed here for simplicity as "non-neutralizing" anti-ADM antibody or antibody fragment, that e.g. blocks the bioactivity of ADM to less than 80 %, is defined as - a molecule or molecules binding to ADM, which upon addition to a culture of an eukaryotic cell line, which expresses functional human recombinant ADM receptor composed of CRLR (calcitonin receptor like receptor) and RAMP3 (receptor-activity modifying protein 3), reduces the amount of cAMP produced by the cell line through the action of parallel added human synthetic ADM peptide, wherein said added human synthetic ADM is added in an amount that in the absence of the non-neutralizing antibody to be analyzed, leads to half- maximal stimulation of cAMP synthesis, wherein the reduction of cAMP by said molecule(s) binding to ADM takes place to an extent, which is not more than 80%, even when the non-neutralizing molecule(s) binding to ADM to be analyzed is added in an amount, which is 10-fold more than the amount, which is needed to obtain the maximal reduction of cAMP synthesis obtainable with the non-neutralizing antibody to be analyzed. The same definition applies to the other ranges; 95%, 90%, 50% etc.

In a specific embodiment according to the present invention an anti-ADM antibody or an anti-ADM antibody fragment is used, wherein said antibody or antibody fragment blocks the bioactivity of ADM to less than 80 %, preferably less than 50% (of baseline values). This is in the sense of blocking the circulating ADM of not more than 80% or not more than 50%, respectively. It has been understood that said limited blocking of the bioactivity of ADM occurs even at excess concentration of the antibody or antibody fragment, meaning an excess of the antibody or antibody fragment in relation to ADM. Said limited blocking is an intrinsic property of the ADM binder itself. This means that said antibody or antibody fragment have a maximal inhibition of 80% or 50%, respectively. By implication, this means that 20% or 50% residual ADM bioactivity remains present although appropriate amounts or excess amounts of antibody, antibody fragment or non-Ig scaffold are administered, respectively.

In a preferred embodiment said anti-ADM antibody or anti-ADM antibody fragment would block the bioactivity of ADM at least 5 %. By implication, this means residual 95% circulating ADM bioactivity remains present. This is the lower threshold of bioactivity remaining after administration of said anti-ADM antibody or anti-ADM antibody fragment. The bioactivity is defined as the effect that a substance takes on a living organism or tissue or organ or functional unit in vivo or in vitro (e.g., in an assay) after its interaction. In case of ADM bioactivity this may be the effect of ADM in a human recombinant ADM receptor cAMP functional assay. Thus, according to the present invention bioactivity is defined via an ADM receptor cAMP functional assay. The following steps may be performed in order to determine the bioactivity of ADM in such an assay:
- Dose response curves are performed with ADM in said human recombinant ADM receptor cAMP functional assay.
- The ADM-concentration of half-maximal cAMP stimulation may be calculated.
- At constant half-maximal cAMP-stimulating ADM-concentrations dose response curves (up to 100 µl final concentration) are performed by an ADM stabilizing antibody or an ADM stabilizing antibody fragment, respectively.

A maximal (at maximal dose) inhibition by said ADM stabilizing antibody of 50% means that said ADM antibody or said ADM antibody fragment, respectively, blocks the bioactivity to 50% of baseline values. A maximal inhibition in said ADM bioassay of 80% means that said anti-ADM antibody or said anti-ADM antibody fragment, respectively, blocks the bioactivity of ADM to 80%. This is in the sense of blocking the ADM bioactivity to not more than 80%.

In a preferred embodiment a modulating anti-ADM antibody or a modulating anti-ADM antibody fragment is used. A "modulating" anti-ADM antibody or a modulating anti-ADM antibody fragment is an anti-ADM antibody or an anti-ADM antibody fragment that enhances the half-life (t half retention time) of ADM in serum, blood, plasma at least 10 %, preferably at least, 50 %, more preferably >50 %, most preferably >100 % and blocks the bioactivity of ADM to less than 80 %, preferably less than 50 % and wherein said anti-ADM antibody or anti-ADM antibody fragment would block the bioactivity of ADM at least 5 %. These values related to half-life and blocking of bioactivity have to be understood in relation to the before-mentioned assays in order to determine these values. This is in the sense of blocking the circulating ADM of not more than 80% or not more than 50%, respectively. This means 20% residual ADM bioactivity remains present, or 50% residual ADM bioactivity remains present, respectively. Such a modulating anti-ADM antibody or a modulating anti-ADM antibody fragment offers the advantage that the dosing of the administration is facilitated. The combination of partially blocking or partially reducing ADM bioactivity and increase of the in vivo half-life (increasing the ADM bioactivity) leads to beneficial simplicity of anti-ADM antibody or an anti-ADM antibody fragment dosing. In a situation of excess endogenous ADM, the activity lowering effect is the major impact of the antibody or fragment, limiting the (negative) effect of ADM. In case of low or normal endogenous ADM concentrations, the biological effect of anti- ADM antibody or an anti-ADM antibody fragment is a combination of lowering (by partially blocking) and increase by increasing the ADM half-life. Thus, the non-neutralizing and modulating ADM antibody or ADM antibody fragment acts like an ADM bioactivity buffer in order to keep the bioactivity of ADM within a certain physiological range.

The anti-ADM antibody or anti-ADM antibody fragment according to the present invention exhibits an affinity towards human ADM that the affinity constant is greater than 10⁻⁷ M, preferred 10⁻⁸ M, preferred affinity is greater than 10⁻⁹ M, most preferred higher than 10⁻¹⁰ M. A person skilled in the art knows that it may be considered to compensate lower affinity by applying a higher dose of compounds and this measure would not lead out-of-the-scope of the invention. The affinity constants may be determined according to the method as described in Example 1 of WO 2013/072513.

It should be emphasized that the term "ADM binding protein" comprises ADM-binding-protein-1 (complement factor H). However, said ADM binding protein by definition pursuant to the invention is neither a non-neutralizing anti-ADM antibody/antibody fragment nor a modulating anti-ADM antibody/antibody fragment. Subject of the present invention is further an anti-ADM antibody or an anti-ADM antibody fragment for use in therapy of acute disease or acute condition of a patient according to the present invention, wherein said antibody or antibody fragment scaffold may be used in combination with further active ingredients.

In a specific embodiment, an assay is used for determining the level of pro-Adrenomedullin or a fragment thereof, wherein said level of pro-Adrenomedullin or a fragment thereof is selected from the group consisting of PAMP (SEQ ID No. 3), MR-proADM (SEQ ID No. 4), ADM-NH₂ (SEQ ID No. 5), ADM-Gly (SEQ ID No. 6) and CT-proADM (SEQ ID No. 7) and wherein such assay is a sandwich assay, preferably a fully automated assay.

In one embodiment of the invention, it may be a so-called POC-test (point-of-care) that is a test technology, which allows performing the test within less than 1 hour near the patient without the requirement of a fully automated assay system. One example for this technology is the immunochromatographic test technology.

In one embodiment of the diagnostic method such an assay is a sandwich immunoassay using any kind of detection technology including but not restricted to enzyme label, chemiluminescence label, electrochemiluminescence label, preferably a fully automated assay. In one embodiment of the diagnostic method such an assay is an enzyme labeled sandwich assay. Examples of automated or fully automated assay comprise assays that may be used for one of the following systems: Roche Elecsys^{®}, Abbott Architect^{®}, Siemens Centauer^{®}, Brahms Kryptor^{®}, BiomerieuxVidas^{®}, Alere Triage^{®}.

A variety of immunoassays are known and may be used for the assays and methods of the present invention, these include: radioimmunoassays ("RIA"), homogeneous enzyme-multiplied immunoassays ("EMIT"), enzyme linked immunoadsorbent assays ("ELISA"), apoenzyme reactivation immunoassay ("ARIS"), dipstick immunoassays and immunochromatography assays.

In a specific embodiment, at least one of said two binders is labeled in order to be detected.

The following embodiments are subject of the present invention:
1. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of Pro-Adrenomedullin (pro-ADM) or fragments thereof above a predetermined threshold in a sample of bodily fluid of said patient, wherein said Pro-Adrenomedullin or fragment is selected from the group comprising Pro-Adrenomedullin according to SEQ ID No. 1 or PAMP according to SEQ ID No.: 3 or MR-proADM according to SEQ ID No.: 4 or mature ADM-NH2 (bio-ADM) according to SEQ ID No.: 5 or ADM-Gly according to SEQ ID No.: 6 or CT-proADM according to SEQ ID No.: 7.
2. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to embodiment 1, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of pro-Adrenomedullin or fragments thereof above a predetermined threshold in a sample of bodily fluid of said patient.
3. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, according to embodiment 1 and 2, and wherein said antibody or antibody fragment is administered to a patient with a level of pro-Adrenomedullin or fragments thereof above a predetermined threshold which is the 95th percentile of the normal range of pro-Adrenomedullin or fragments thereof of a healthy subject, more preferred which is the 96th percentile of the normal range of bio-ADM of a healthy subject, even more preferred which is the 97th percentile of the normal range of a healthy subject, wherein said sample of bodily fluid is selected from the group comprising whole blood, plasma, and serum, in particular blood plasma.
4. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, according to embodiment 1 to 3, wherein said antibody or antibody fragment is administered to a patient with a level of pro-Adrenomedullin or fragments thereof above a predetermined threshold, which is for the amount of bio-ADM in a sample of bodily fluid ≥ 43 pg/ml, wherein said sample of bodily fluid is selected from the group comprising whole blood, plasma, and serum, in particular blood plasma.
5. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to embodiment 1 to 4 and wherein said antibody or antibody fragment is administered to a patient having a level of pro-Adrenomedullin or fragments thereof, wherein such level is determined from a sample of bodily fluid taken pre-, intra- or post-operatively.
6. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to embodiment 1 to 5 and wherein said antibody or antibody fragment is administered to a patient having a level of pro-Adrenomedullin or fragments thereof, wherein the level of pro-Adrenomedullin or fragments thereof is determined in a bodily fluid obtained from said patient is measured with an immunoassay.
7. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to any of embodiments 1 to 6, wherein said antibody or antibody fragment may be administered in a dose of at least 0.5 mg / kg body weight, particularly at least 1.0 mg/kg body weight, more particularly, from 1.0 to 20.0 mg/kg body weight, e.g., from 2.0 to 10 mg/kg body weight, from 2.0 to 8.0 mg/kg body weight, or from 2.0 to 5.0 mg/kg body weight.
8. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to any of embodiments 1 to 7, wherein said antibody or antibody fragment is a human monoclonal antibody or a fragment that binds to the N-terminal region (aa 1-21) of ADM (SEQ ID No. 2) or an antibody fragment thereof wherein the heavy chain comprises the sequences:
   CDR1: SEQ ID NO: 8
      GYTFSRYW
   CDR2: SEQ ID NO: 9
      ILPGSGST
   CDR3: SEQ ID NO: 10
      TEGYEYDGFDY
   and wherein the light chain comprises the sequences:
   CDR1: SEQ ID NO: 11
      QSIVYSNGNTY
   CDR2:
      RVS
   CDR3: SEQ ID NO: 12
      FQGSHIPYT.
9. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, according to any of embodiments 1 to 8, wherein said antibody or fragment comprises the following sequence as a heavy chain: 
SEQ ID NO: 22

QVQLVQSGAEVKKPGSSVKVSCKASGYTFSRYWIEWVRQAPGQGLEWIGEILPG
SGSTNYNQKFQGRVTITADTSTSTAYMELSSLRSEDTAVYYCTEGYEYDGFDYW
GQGTTVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNK
 
ALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHY
TQKSLSLSPGK
   or a sequence that is > 95% identical to it,
   and comprises the following sequence as a light chain: 
SEQ ID NO: 23

DVVLTQSPLSLPVTLGQPASISCRSSQSIVYSNGNTYLEWYLQRPGQSPRLLIYRVS
NRFSGVPDRFSGSGSGTDFTLKISRVEAEDVGVYYCFQGSHIPYTFGGGTKLEIKR
TVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
   or a sequence that is > 95% identical to it.
10. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to any of embodiments 1 to 9, wherein said antibody or antibody fragments are administered orally, intravenously, subcutaneously, intraarterially, intramuscularly, intracardially, intraspinally, intrathoracically, intraperitoneal, intraventricular, sublingually, transdermal, and/or via inhalation.

### Description of the figures

**Figure 1****:** Comparison of the illness score over 540 minutes after application of different doses of Adrecizumab (0.5, 2 and 8 mg/kg) and a Placebo. Treatment start was 180 minutes after LPS challenge and the duration of treatment 60 min.
**Figure 2****:** Distribution of the illness scores by treatment arm at T270 (time point 270 min) and T300 (time point 300 min), respectively (Fig. 2 A) and B).
**Figure 3****:** The contribution of single illness score components to the effect; A) nausea, B) headache, C) muscle aches, D) back pain, and E) shivering. All symptoms shown contribute significantly to the effect.
**Figure 4****:** An alternative evaluation is shown. Here, the Sickness score was evaluated using the single question regarding the overall sickness, on a scale from 0 to 10.
**Figure 5****:** Boxplots comparing pre- and post-operative bio-ADM values. Paired two-tailed t-test was performed and p values between timepoints are shown; black dashed line: bio-ADM cut-off at 51.35 pg/mL; grey dashed line: bio-ADM cut-off at 43 pg/mL.
**Figure 6****:** Receiver operating characteristic (ROC) curve showing the predictive performance of maximum post-operative bio-ADM levels to identify patients who require chest tube placement for pleural effusion; AUC=0.819.
**Figure 7****:** Correlation of bio-ADM levels at timepoint 2 with length of cardiopulmonary bypass (min); n=73; Spearman's r=0.268; p<0.022.
**Figure 8****:** Correlation of maximum post-operative bio-ADM levels with length of intubation; n=73; Spearman's r=0.257; p<0.028.
**Figure 9****:** Correlation of maximum post-operative bio-ADM values (pg/mL) with length of catecholamines (hours); dashed line: cut-off at 43 pg/mL; n=73; Spearman's r=0.415; p<0.001.
**Figure 10****:** Correlation of maximum post-operative bio-ADM values (pg/mL) with fluid balance (ml/kg); dashed line: cut-off at 43 pg/mL; n=67; Spearman's r=0.372; p<0.001.
**Figure 11****:** Boxplots comparing bio-ADM values in children who received (yes) and did not receive (no) a chest tube for pleural effusion drainage. The bio-ADM values measured most closely to chest tube placement (yes-group) were compared with the maximum post-operative bio-ADM values in children without drainage (no-group). Mann-Whitney-U-test was performed and p-value between the two groups is shown.
**Figure 12****:** 9-day Kaplan-Meier survival curves for the prediction of chest tube placement for pleural effusion in observation period are shown for patients with maximum post-operative bio-ADM levels above and below the cut-off of 43 pg/mL; Log-Rank test was performed. P<0.001.
**Figure 13****:** 9-day Kaplan-Meier survival curves for the prediction of chest tube placement for pleural effusion in observation period are shown for patients with maximum post-operative bio-ADM levels above or below the cut-off of 51.35 pg/mL; Log-Rank test was performed. P<0.001.
**Figure 14 A-E****:** Clinical correlation: bio-ADM and pleural effusions requiring drainage. Shown are results obtained at different time points for the subpopulation of patients not having received dexamethasone.
**Figure 15****:** Boxplots comparing bio-ADM levels at timepoint 5 in patients with no infection, infection and sepsis. TP 5 was chosen because it is the blood sample taken most closely to start of antibiotic treatment. n=53; some TP5 blood samples are missing; Mann-Whitney U test was performed and p values between groups are shown [no infection and infection; infection ad sepsis and no infection and sepsis].
**Figure 16****:** Boxplots comparing bio-ADM levels in patients with (yes) and without (no) pre-surgery dexamethasone treatment. Mann-Whitney-U test was performed; significant p-values are shown.
**Figure 17****:** Box plots comparing maximum bio-Adrenomedullin levels in patients with pre surgery dexamethasone, post-surgery cortisone, pre- and post-surgery cortisone, and no cortisone. Mann-Whitney U test was performed. No significant differences were found.

### Examples

### Example 1: Generation of Antibodies and determination of their affinity constants

Several human and murine antibodies were produced and their affinity constants were determined (see Table 1).

### Peptides / conjugates for Immunization:

Peptides for immunization were synthesized, see Table 1, (JPT Technologies, Berlin, Germany) with an additional N-terminal Cysteine (if no Cysteine is present within the selected ADM-sequence) residue for conjugation of the peptides to Bovine Serum Albumin (BSA). The peptides were covalently linked to BSA by using Sulfolink-coupling gel (Perbio Science, Bonn, Germany). The coupling procedure was performed according to the manual of Perbio.

The murine antibodies were generated according to the following method:
A Balb/c mouse was immunized with 100µg Peptide-BSA-Conjugate at day 0 and 14 (emulsified in 100µl complete Freund's adjuvant) and 50µg at day 21 and 28 (in 100µl incomplete Freund's adjuvant). Three days before the fusion experiment was performed, the animal received 50µg of the conjugate dissolved in 100µl saline, given as one intraperitoneal and one intra-venous injection.

Splenocytes from the immunized mouse and cells of the myeloma cell line SP2/0 were fused with 1ml 50% polyethylene glycol for 30s at 37°C. After washing, the cells were seeded in 96-well cell culture plates. Hybrid clones were selected by growing in HAT medium (RPMI 1640 culture medium supplemented with 20% fetal calf serum and HAT-Supplement). After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting, the selected cultures were cloned and recloned using the limiting-dilution technique and the isotypes were determined (see also Lane. R.D. 1985. J. Immunol. Meth. 81: 223-228*;* Ziegler et al. 1996. Horm. Metab. Res. 28: 11-15).

### Mouse monoclonal antibody production:

Antibodies were produced via standard antibody production methods *(*Marx et al, 1997. Monoclonal Antibody Production, ATLA 25, 121) and purified via Protein A. The antibody purities were > 95% based on SDS gel electrophoresis analysis.

### Human Antibodies:

Human Antibodies were produced by means of phage display according to the following procedure:
The human naive antibody gene libraries HAL7/8 were used for the isolation of recombinant single chain F-Variable domains (scFv) against ADM peptide. The antibody gene libraries were screened with a panning strategy comprising the use of peptides containing a biotin tag linked via two different spacers to the ADM peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatant from the cultivation of these clonal strains has been directly used for an antigen ELISA testing (see also Hust et al. 2011. Journal of Biotechnology 152, 159-170; Schütte et al. 2009. PLoS One 4, e6625).

Positive clones have been selected based on positive ELISA signal for antigen and negative for streptavidin coated micro titer plates. For further characterizations the scFv open reading frame has been cloned into the expression plasmid pOPE107 (Hust et al. 2011. Journal of Biotechnology 152, 159 170), captured from the culture supernatant via immobilized metal ion affinity chromatography and purified by a size exclusion chromatography.

### Affinity Constants:

To determine the affinity of the antibodies to ADM, the kinetics of binding of ADM to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare) (Lorenz et al. 2011. Antimicrob Agents Chemother. 55(1): 165-173).

The monoclonal antibodies were raised against the below depicted ADM regions of human and murine ADM, respectively. The following table represents a selection of obtained antibodies used in further experiments. Selection was based on target region:

**Table 1: Selection of obtained antibodies used in further experiments.**

| Sequence Number | Antigen/Immunogen | ADM Region | Designation | Affinity constants Kd (M) |
|---|---|---|---|---|
| SEQ ID: 2 | YRQSMNNFQGLRSFGCRFGTC | 1-21 | NT-H | 5.9 × 10⁻⁹ |
| SEQ ID: 24 | CTVQKLAHQIYQ | 21-32 | MR-H | 2 × 10⁻⁹ |
| Amidated SEQ ID: 25 (with additional Cysteine at N-terminus) | C-APRSKISPQGY-NH₂ | C-42-52 | CT-H | 1.1 × 10⁻⁹ |
| SEQ ID: 26 | YRQSMNQGSRSNGCRFGTC | 1-19 | NT-M | 3.9 × 10⁻⁹ |
| SEQ ID: 27 | CTFQKLAHQIYQ | 19-31 | MR-M | 4.5 × 10⁻¹⁰ |
| Amidated SEQ ID: 28 (with additional Cysteine at N-terminus) | C-APRNKISPQGY-NH₂ | C-40-50 | CT-M | 9 × 10⁻⁹ |

### Generation of antibody fragments by enzymatic digestion:

The generation of Fab and F(ab)2 fragments was done by enzymatic digestion of the murine full length antibody NT-M. Antibody NT-M was digested using a) the pepsin-based F(ab)2 Preparation Kit (Pierce 44988) and b) the papain-based Fab Preparation Kit (Pierce 44985). The fragmentation procedures were performed according to the instructions provided by the supplier. Digestion was carried out in case of F(ab)2-fragmentation for 8h at 37°C. The Fab-fragmentation digestion was carried out for 16h, respectively.

### Procedure for Fab Generation and Purification:

The immobilized papain was equilibrated by washing the resin with 0.5 ml of Digestion Buffer and centrifuging the column at 5000 × g for 1 minute. The buffer was discarded afterwards. The desalting column was prepared by removing the storage solution and washing it with digestion buffer, centrifuging it each time afterwards at 1000 × g for 2 minutes. 0.5ml of the prepared IgG sample were added to the spin column tube containing the equilibrated Immobilized Papain. Incubation time of the digestion reaction was done for 16h on a tabletop rocker at 37°C. The column was centrifuged at 5000 × g for 1 minute to separate digest from the Immobilized Papain. Afterwards the resin was washed with 0.5ml PBS and centrifuged at 5000 × g for 1 minute. The wash fraction was added to the digested antibody that the total sample volume was 1.0ml. The NAb Protein A Column was equilibrated with PBS and IgG Elution Buffer at room temperature. The column was centrifuged for 1 minute to remove storage solution (contains 0.02% sodium azide) and equilibrated by adding 2ml of PBS, centrifuge again for 1 minute and the flow-through discarded. The sample was applied to the column and resuspended by inversion. Incubation was done at room temperature with end-over-end mixing for 10 minutes. The column was centrifuged for 1 minute, saving the flow-through with the Fab fragments. (References: Coulter and Harris 1983. J. Immunol. Meth. 59. 199-203*.;* Lindner et al. 2010. Cancer Res. 70, 277-87*;* Kaufmann et al. 2010. PNAS. 107, 18950-5*.;* Chen et al. 2010. PNAS. 107, 14727-32*;* Uysal et al. 2009 J. Exp. Med. 206, 449-62*;* Thomas et al. 2009. J. Exp. Med. 206, 1913-27*;* Kong et al. 2009 J. Cell Biol. 185, 1275-840).

### Procedure for generation and purification of F(ab')2 Fragments:

The immobilized Pepsin was equilibrated by washing the resin with 0.5 ml of Digestion Buffer and centrifuging the column at 5000 × g for 1 minute. The buffer was discarded afterwards. The desalting column was prepared by removing the storage solution and washing it with digestion buffer, centrifuging it each time afterwards at 1000 × g for 2 minutes. 0.5ml of the prepared IgG sample were added to the spin column tube containing the equilibrated Immobilized Pepsin. Incubation time of the digestion reaction was done for 16h on a tabletop rocker at 37°C. The column was centrifuged at 5000 × g for 1 minute to separate digest from the Immobilized Papain. Afterwards the resin was washed with 0.5mL PBS and centrifuged at 5000 × g for 1 minute. The wash fraction was added to the digested antibody that the total sample volume was 1.0ml. The NAb Protein A Column was equilibrated with PBS and IgG Elution Buffer at room temperature. The column was centrifuged for 1 minute to remove storage solution (contains 0.02% sodium azide) and equilibrated by adding 2mL of PBS, centrifuge again for 1 minute and the flow-through discarded. The sample was applied to the column and resuspended by inversion. Incubation was done at room temperature with end-over-end mixing for 10 minutes. The column was centrifuged for 1 minute, saving the flow-through with the Fab fragments. (References: Mariani et al. 1991. Mol. Immunol. 28: 69-77*;* Beale 1987. Exp Comp Immunol 11:287-96*;* Ellerson et al. 1972. FEBS Letters 24(3):318-22*;* Kerbel and Elliot 1983. Meth Enzymol 93:113-147*;* Kulkarni et al. 1985. Cancer Immunol Immunotherapy 19:211-4*;* Lamoyi 1986. Meth Enzymol 121:652-663*;* Parham et al. 1982. J Immunol Meth 53:133-73*;* Raychaudhuri et al. 1985. Mol Immunol 22(9):1009-19*;* Rousseaux et al. 1980. Mol Immunol 17:469-82*;* Rousseaux et al. 1983. J Immunol Meth 64:141-6*;* Wilson et al. 1991. J Immunol Meth 138:111-9).

### NT-H-Antibody Fragment Humanization:

The antibody fragment was humanized by the CDR-grafting method (Jones et al. 1986. Nature 321, 522-525).

The following steps were executed to achieve the humanized sequence:
- Total RNA extraction: Total RNA was extracted from NT-H hybridomas using the Qiagen kit.
- First-round RT-PCR: QIAGEN^{®} OneStep RT-PCR Kit (Cat No. 210210) was used. RT-PCR was performed with primer sets specific for the heavy and light chains. For each RNA sample, 12 individual heavy chain and 11 light chain RT-PCR reactions were set up using degenerate forward primer mixtures covering the leader sequences of variable regions. Reverse primers are located in the constant regions of heavy and light chains. No restriction sites were engineered into the primers.
- Reaction Setup: 5x QIAGEN^{®} OneStep RT-PCR Buffer 5.0 µl, dNTP Mix (containing 10 mM of each dNTP) 0.8 µl, Primer set 0.5 µl, QIAGEN^{®} OneStep RT-PCR Enzyme Mix 0.8 µl, Template RNA 2.0 µl, RNase-free water to 20.0 µl, Total volume 20.0 µl PCR condition: Reverse transcription: 50°C, 30 min; Initial PCR activation: 95°C, 15 min Cycling: 20 cycles of 94°C, 25 sec; 54°C, 30 sec; 72°C, 30 sec; Final extension: 72°C, 10 min Second-round semi-nested PCR: The RT-PCR products from the first-round reactions were further amplified in the second-round PCR. 12 individual heavy chain and 11 light chain RT-PCR reactions were set up using semi-nested primer sets specific for antibody variable regions.
- Reaction Setup: 2x PCR mix 10 µl; Primer set 2 µl; First-round PCR product 8 µl; Total volume 20 µl; Hybridoma Antibody Cloning Report PCR condition: Initial denaturing of 5 min at 95°C; 25 cycles of 95°C for 25 sec, 57°C for 30 sec, 68°C for 30 sec; Final extension is 10 min 68°C
- After PCR is finished, run PCR reaction samples onto agarose gel to visualize DNA fragments amplified. After sequencing more than 15 cloned DNA fragments amplified by nested RT-PCR, several mouse antibody heavy and light chains have been cloned and appear correct. Protein sequence alignment and CDR analysis identifies one heavy chain and one light chain. As the amino acids on positions 26, 40 and 55 in the variable heavy chain and amino acid on position 40 in the variable light are critical to the binding properties, they may be reverted to the murine original. The resulting candidates are depicted below. (Padlan 1991. Mol. Immunol. 28. 489-498*;* Harris and Bajorath. 1995. Protein Sci. 4. 306-310).

Annotation for the antibody fragment sequences (SEQ ID No.: 13 to 23): bold and underlined are the CDR 1, 2, 3 in numeric order from the N-terminus to the C-terminus; italic are constant regions; hinge regions are highlighted with bold, underlined letters.
SEQ ID No.: 13 (AM-VH-C) 
QVQLQQSGAELMKPGASVKISCKATGYTFSRYWIEWVKQRPGHGLEWIGEILPGSG
STNYNEKFKGKATITADTSSNTAYMQLSSLTSEDSAVYYCTEGYEYDGFDYWGQGTTLT
 
VSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPK
SEQ ID No.: 14 (AM-VH1) 
QVQLVQSGAEVKKPGSSVKVSCKASGYTFSRYWISWVRQAPGQGLEWMGRILPGS
GSTNYAQKFQGRVTITADESTSTAYMELSSLRSEDTAVYYCTEGYEYDGFDYWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPK
SEQ ID No.: 15 (AM-VH2-E40) 
QVQLVQSGAEVKKPGSSVKVSCKASGYTFSRYWIEWVRQAPGQGLEWMGRILPGS
GSTNYAQKFQGRVTITADESTSTAYMELSSLRSEDTAVYYCTEGYEYDGFDYWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPK
SEQ ID No.: 16 (AM-VH3-T26-E55) 
QVQLVQSGAEVKKPGSSVKVSCKATGYTFSRYWISWVRQAPGQGLEWMGEILPGS
GSTNYAQKFQGRVTITADESTSTAYMELSSLRSEDTAVYYCTEGYEYDGFDYWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPK
SEQ ID No.: 17 (AM-VH4-T26-E40-E55) 
QVQLVQSGAEVKKPGSSVKVSCKATGYTFSRYWIEWVRQAPGQGLEWMGEILPGS
GSTNYAQKFQGRVTITADESTSTAYMELSSLRSEDTAVYYCTEGYEYDGFDYWGQGTTV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPK
SEQ ID No.: 18 (AM-VL-C) 
DVLLSQTPLSLPVSLGDQATISCRSSQSIVYSNGNTYLEWYLQKPGQSPKLLIYRVSN
RFSGVPDRFSGSGSGTDFTLKISRVEAEDLGVYYCFQGSHIPYTFGGGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID No.: 19 (AM-VL1) 
DVVMTQSPLSLPVTLGQPASISCRSSQSIVYSNGNTYLNWFQQRPGQSPRRLIYRVSN
RDSGVPDRFSGSGSGTDFTLKISRVEAEDVGVYYCFQGSHIPYTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID No.: 20 (AM-VL2-E40) 
DVVMTQSPLSLPVTLGQPASISCRSSQSIVYSNGNTYLEWFQQRPGQSPRRLIYRVSN
RDSGVPDRFSGSGSGTDFTLKISRVEAEDVGVYYCFQGSHIPYTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 22 (Adrecizumab/ HAM8101 heavy chain) 
QVQLVQSGAEVKKPGSSVKVSCKASGYTFSRYWIEWVRQAPGQGLEWIGEILPGSG
STNYNQKFQGRVTITADTSTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVL
D  SDGSFFLYSKLTVDKSRWQQGNVFSCSVMHEALHNHYTQKSLSLSPGK
SEQ ID NO: 23 (Adrecizumab/ HAM8101 light chain)

DVVLTQSPLSLPVTLGQPASISCRSSQSIVYSNGNTYLEWYLQRPGQSPRLLIYRVSN
RFSGVPDRFSGSGSGTDFTLKISRVEAEDVGVYYCFQGSHIPYTFGGGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

**Example 2** - A randomized double-blind placebo-controlled phase I study on the safety, tolerability and pharmacokinetics/-dynamics of escalating single intravenous doses of Anti-ADM-antibody (ADRECIZUMAB/ HAM8101) in healthy male subjects during experimental endotoxemia.

### Overall trial design

A randomized, double-blind, placebo-controlled phase I study in healthy male volunteers during experimental endotoxemia with single, escalating per group doses of Adrecizumab administered as *i*.*v*. infusion over a 1-hour period. A continuous LPS (lipopolysaccharide) model was used to induce experimental endotoxemia; administration of LPS in an initial bolus of 1 ng/kg followed by continuous infusion at 1 ng/kg/h for 3 hours. Subject will receive one course of treatment with study medication (Adrecizumab or placebo), 1 hour after start of LPS administration. The model of systemic inflammation has been recently described (Kiers et al. 2017. Scientific Reports 7: 40149).

Adrecizumab was administrated as single infusion over a 1-hour period in a dose of 0.5 mg/kg and was increased up to 2.0 and 8.0 mg/kg in subsequent treatment groups (see Table 2).

**Table 2: Study groups of 8 subjects received treatment by 1-hour infusion.**

| **Group** | **Treatment** | |
|---|---|---|
| Group 1 | 0.5 mg/kg HAM8101 (n=6) | Placebo (n=2) |
| Group 2 | 2.0 mg/kg HAM8101 (n=6) | Placebo (n=2) |
| Group 3 | 8.0 mg/kg HAM8101 (n=6) | Placebo (n=2) |

### Selection of trial population

The study population consisted of healthy young male volunteers. To be included into the trial, subjects had to meet all inclusion criteria and none of the exclusion criteria.

### Inclusion criteria

1. Written informed consent to participate in this trial prior to any study-mandated procedure.
2. Male subjects aged 18 to 35 years inclusive.
3. Subjects have to agree to use a reliable way of contraception with their partners from study entry until 3 months after study drug administration.
4. BMI between 18 and 30 kg/m², with a lower limit of body weight of 50 kg and an upper limit of 100 kg.
5. Healthy as determined by medical history, physical examination, vital signs, 12-lead electrocardiogram, and clinical laboratory parameters.

### Exclusion criteria

1. Unwillingness to abstain from any medication, recreational drugs or anti-oxidant vitamin supplements during the course of the study and within 7 days prior to the treatment day.
2. Unwillingness to abstain from smoking, or alcohol within 1 day prior to the treatment day.
3. Previous participation in a trial where LPS was administered.
4. Surgery or trauma with significant blood loss or blood donation within 3 months prior to the treatment day.
5. History, signs or symptoms of cardiovascular disease, in particular:
   - History of frequent vasovagal collapse or of orthostatic hypotension
   - Resting pulse rate ≤45 or ≥100 beats /min
   - Hypertension (RR systolic >160 or RR diastolic >90 mmHg)
   - Hypotension (RR systolic <100 or RR diastolic <50 mmHg)
   - Conduction abnormalities on the ECG consisting of a 1st degree atrioventricular block or a complex bundle branch block
   - Any chronic cardiac arrhythmias (except PAC's, PVC's)
6. Renal impairment: plasma creatinine >120 µmol/L
7. Liver function tests (alkaline phosphatase, AST, ALT and/or γ-GT) above 2x the upper limit of normal.
8. History of asthma
9. Atopic constitution
10. CRP above 2x the upper limit of normal, or clinically significant acute illness, including infections, within 2 weeks before the treatment day.
11. Treatment with investigational drugs or participation in any other clinical trial within 30 days prior to the Treatment day.
12. Known or suspected of not being able to comply with the trial protocol.
13. Known hypersensitivity to any excipients of the drug formulations used.
14. Inability to personally provide written informed consent (e.g. for linguistic or mental reasons) and/or take part in the study.

### Lipopolysaccharide (LPS) from Escherichia coli Type 0113

To achieve a controlled inflammatory state, subjects received lipopolysaccharide (LPS, U.S. reference endotoxin [Escherichia coli O:113, List Biological Laboratories Inc., Campbell, California, US]) intravenously.

### Examination hierarchy and time windows

The pre-dose blood samples were drawn on T=0 (baseline). Vital signs were measured after 5 min of resting in supine or semi-supine position. Schedule for blood samples and control visits (see table 3 for baseline information):
- On the treatment day (Day 0), blood samples had to be taken at scheduled times ± 5 min.
- After 24 hours (Day 1), the follow-up visit and blood withdrawal had to be planned at ± 1 hour after IMP administration.
- On day 7, the follow-up visit could be planned at any time, ± 1 day.
- On day 14 and 28, the follow-up visit could be planned at any time, ±2 days.
- On day 60 and 90, the follow-up visit could be planned at any time, ± 3 days.

### Laboratory monitoring

The following hematology parameters were analyzed: Hemoglobin, Hematocrit, RBC, MCV, MCH, MCHC, WBC, Platelets, Differential blood count.

Blood samples were collected in serum separation tubes and the following biochemistry parameters were analyzed: CRP, AF, ALT, AST, γ-GT, Creatine kinase, LDH, Urea-N, Creatinine, Sodium, Potassium, PT and APTT

Primary endpoint variables are: Adverse Events; vital signs during the first 10 hours after Adrecizumab administration and at follow-up periods (T=24 hours, T=7 days, T=14 days, T=28 days, T=60 days, T=90 days), e.g. heart rate, blood pressure, oxygen saturation, temperature; Local tolerability at site of i.v. infusion; safety laboratory parameters (Hb, Ht, leukocytes, thrombocytes, leukocyte differential blood count, sodium, potassium, creatinine, urea, alkaline phosphatase, ALT, AST, GGT, CK, CRP, PT, APTT); 12-lead electrocardiogram (ECG), 2 and 8 hours after Adrecizumab administration.

Secondary endpoints are: Pharmacokinetics of Adrecizumab during experimental endotoxemia (including AUC, Cmax, Terminal T1/2, Cl, V), plasma levels of inflammatory mediators on the endotoxemia day (including but not limited to TNFα, IL-6, IL-8, IL-10); Symptom Illness score; Optional: Kidney damage markers (including but not limited to creatinine clearance, pro-Enkephalin and KIM-1).

**Table 3: Baseline characteristics per group (mean ± SD)**

| | Placebo | 0.5 mg/kg | 2.0 mg/kg | 8.0 mg/kg | P-value ^{∗} |
|---|---|---|---|---|---|
| Age | 22.0 ± 1.1 | 22.0 ± 2.8 | 23.3 ± 2.5 | 22.5 ± 2.7 | 0.745 |
| BMI | 23.7 ± 1.3 | 23.3 ± 1.5 | 24.7 ± 1.9 | 25.6 ± 3.0 | 0.263 |

| | | | | | |
|---|---|---|---|---|---|
| ^{∗} Tested with one-way ANOVA | | | | | |

No Serious Adverse Events (SAEs) or Suspected Unsuspected Serious Adverse Reactions (SUSARs) were observed during the complete duration of the study, including the 90 day follow-up period.

### Results

The Illness Score drops quicker under Adrecizumab treatment compared to placebo.

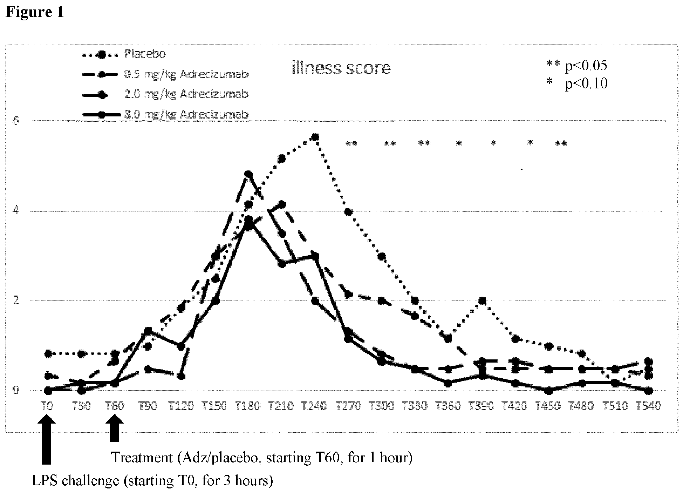

Both, 2 and 8 mg/kg have a significantly lower illness score than placebo between the time points at 210 min (T210) to 450 min (T450) (p=0.011 and 0.005, respectively; GLM repeated measures) (Fig.1).

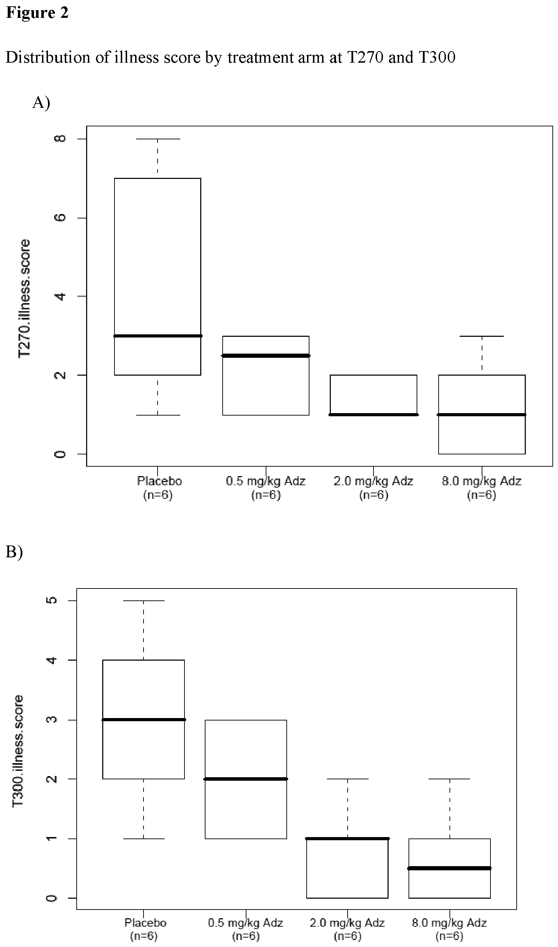

Figure 2 shows the distribution of the illness scores by treatment arm at T270 (time point 270 min) and T300 (time point 300 min), respectively (Fig. 2 A) and B)) supporting the surprisingly beneficial effect of the antibody of the present invention.

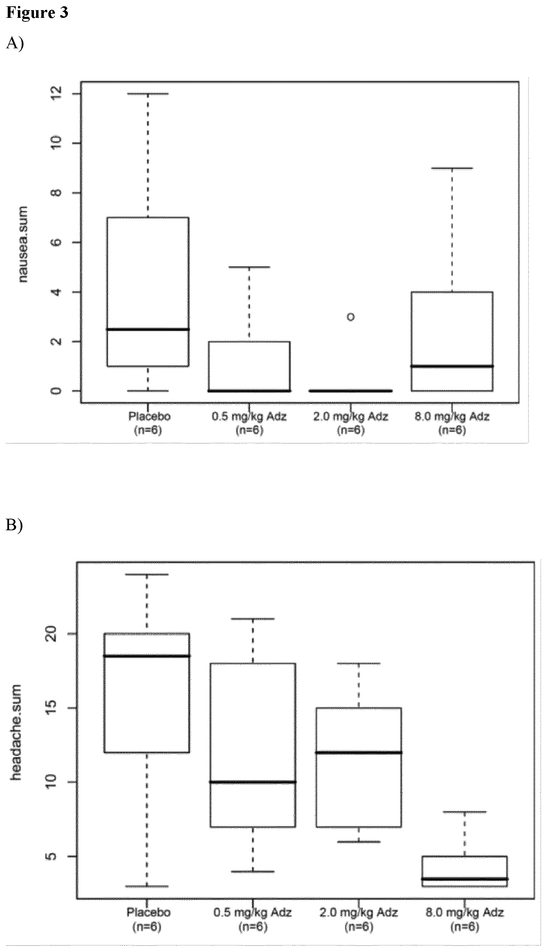

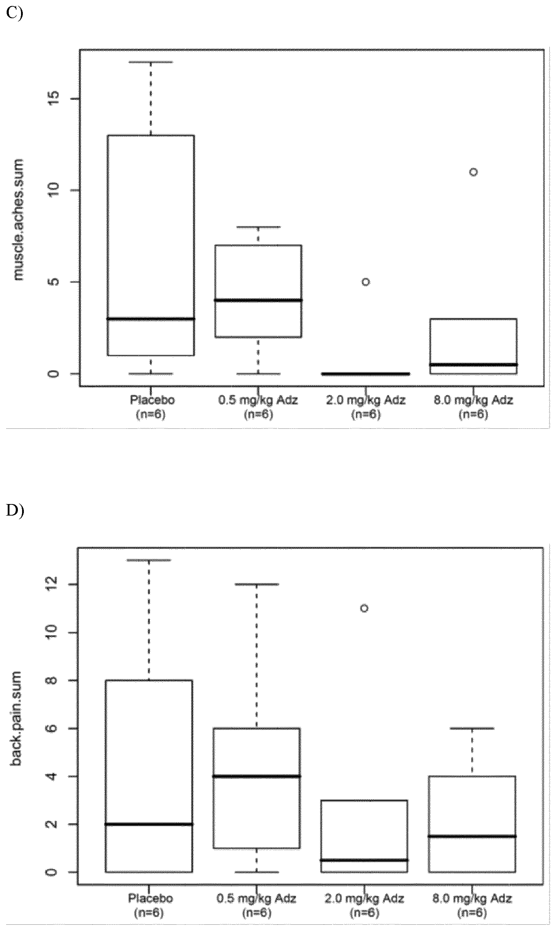

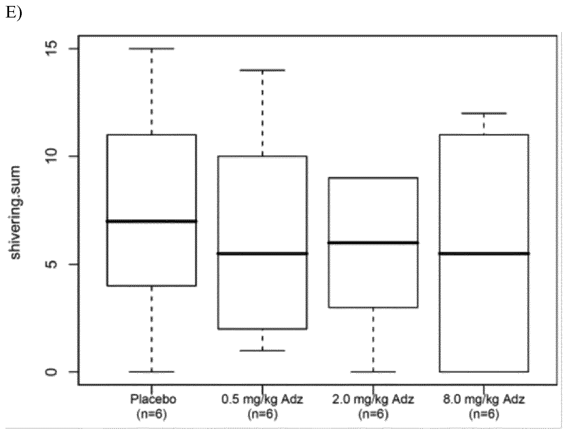

The contribution of single illness score components to the effect is illustrated in Figure 3: A) nausea, B) headache, C) muscle aches, D) back pain, and E) shivering. All symptoms shown contribute significantly to the effect. Vomiting occurred only in two patients once each and could not be analyzed.

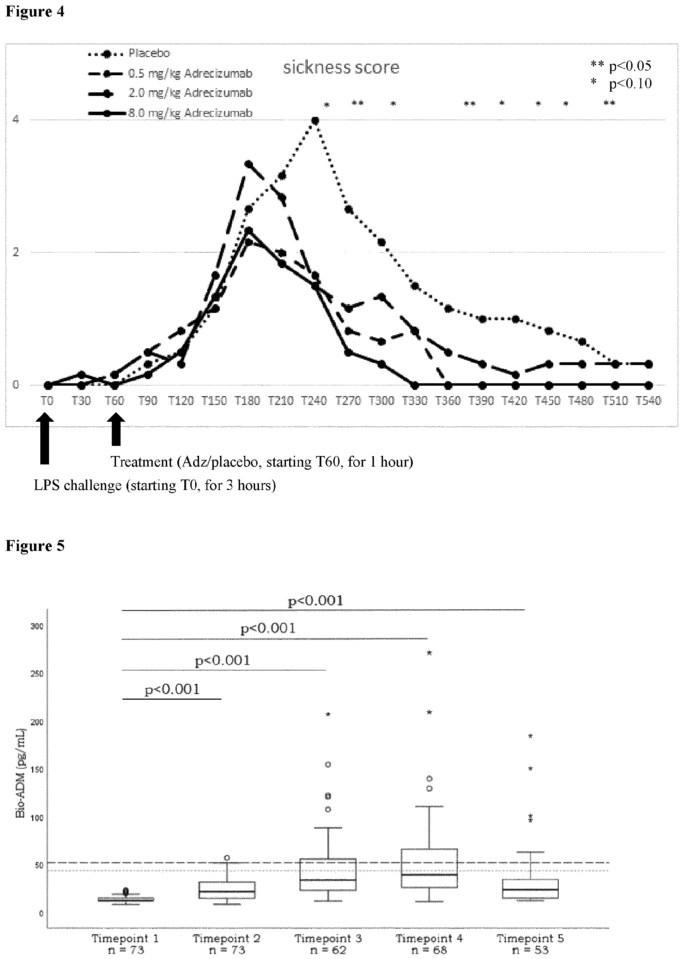

Figure 4 shows an alternative evaluation. Here another score, the Sickness score was evaluated using the single question regarding the overall sickness, on a scale from 0 to 10. Again, the sickness score drops quicker in anti-ADM-antibody (Adrecizumab)-treated patients.

### Example 3: Patient cohorts and methods

### Study design

This was a prospective single center study conducted over two time periods: 08/2019 and 09/2020 to 01/2021. The study protocol was approved by the ethical committee of "Technical University Munich" (protocol number 190/19S from 07/15/2019)

### Study population

This study enrolled 73 pediatric patients (41 males and 32 females) with congenital heart disease and median (IQR) age 21 (4 to 46.5) months admitted to the hospital at the respective timepoints who had heart surgery on CPB. Pre-operative diagnosis included various types of congenital heart disease. Patient's characteristics are shown in Table . Written informed consent was obtained from children's parents or legal guardians.

Exclusion criteria included pre-operative need of catecholamines, organ insufficiency or electrolyte and protein fluctuation as well as missing parent's consent and pre-surgery body weight < 3000g.

The primary endpoint was a net increase in bio-ADM levels after surgery under extracorporeal circulation. Secondary endpoints were correlation of bio-ADM with clinical outcome such as mortality, length of ICU stay, length of hospital stay and intubation time as well as correlation of bio-ADM with clinical signs of systemic inflammation and endothelial dysfunction. To quantify the degree of endothelial dysfunction post-operative fluid balance (within first 24 hours post-surgery), length of catecholamine use, body weight and occurrence of pleural effusion were analyzed. Tertiary endpoint was to validate the relationship between bio-ADM levels and infection. Patients were divided into groups. Patients who needed antibiotic treatment in the post-operative period were classified as having an infection, patients who additionally fulfilled the SIRS criteria defined by the "International pediatric sepsis consensus conference" (Goldstein, B., et al. (2005) Pediatr Crit Care Med 6; 2-8) were defined as having sepsis.

### Collection of patient data

Patient demographics and relevant pre-, intra-, and post-operative data were extracted from medical records. Following variables were collected daily over the 7 days study period: vital signs, ventilation status, blood gas analysis, routine laboratory values and vasopressor treatment.

### Sample collection

Blood samples for bio-ADM measurement were taken on five different timepoints (TP) in each patient. Pre-operatively (TP1), intra-operatively before end of cardiopulmonary bypass (TP2) and on three timepoints post-operatively: 6h (TP3), 24h (TP4) and 1 week (TP5) after surgery. Sample volume was calculated based on the patient's body weight. Blood samples were processed and stored at - 20° Celsius to - 80° Celsius in the central laboratory of "German Heart Center Munich". Blinded bio-ADM measurement was performed in a central lab. For details on the immunoassay used see publications by Weber et al. (Weber, J., et al. (2017) J Appl Lab Med 2; 222-33) and Marino et al. (Marino, R., et al. (2014) Critical care 18; R34). The assay's sensitivity is 12 pg/mL. All samples with lower concentrations were determined as 12 pg/mL.

### Statistical analyses

Categorical variables are presented as number and percentage, continuous variables as median and IQR. The nonparametric Spearman correlation coefficient (r) is given to describe association between two continuous variables. Categorial variables were analyzed using nonparametric Mann-Whitney-U test for two variables and Kruskal-Wallis test for more than two variables. Paired two-tailed t-test was used to compare means of continuous variables. For the comparison of bio-ADM levels between children with and without pleural drainage the bio-ADM level which was measured closest in time to chest tube placement and the maximum bio-ADM value in children without drainage was used. A pre-defined bio-ADM cut-off of 43 pg/mL, representing the 99^{th} percentile of the healthy normal distribution (Marino, R., et al. (2014) Critical care 18; R34), was applied on the development of pleural effusion. A receiver operating characteristic (ROC) analysis was performed to identify the optimal Youden cut-off in this cohort which is 51.35 pg/mL. Based on these cut-off values Kaplan-Meier-curves were created for illustrative purposes. A two-sided p-value of 0.05 was considered statistically significant. All analysis were performed using IBM SPSS Statistics version 28 software.

### Results

A total of 73 patients were included in the study. Patient characteristics, intra- and post-operative data are shown in Table 4.

**Table 4: Patient characteristics.**

| Characteristic | | Patients (N=73) |
|---|---|---|
| Sex - no. (%) | | |
| | Male | 41 (56.2) |
| | Female | 32 (43.8) |
| | | |

| Age at the time of surgery (months) - median (IQR) | | 21.0 (4; 46.5) |
|---|---|---|
| | Infant (0 - 12 months) - no. (%) | 33 (45.2) |
| | Toddler + Preschool (1 - 6 yrs.) - no. (%) | 26 (35.6) |
| | School age (6 - 12 years) - no. (%) | 7 (9.6) |
| | Adolescent and Young (12 - 18 years) - no. (%) | 7 (9.6) |
| | | |

| Type of surgery - no. (%) | | |
|---|---|---|
| | Ventricular septal defect repair | (11.0) |
| | Partial cavopulmonary connection (PCPC) | 7 (9.6) |
| | Total cavopulmonary connection (TCPC) | 11 (15.1) |
| | Combined atrioventricular septal defect repair | 7 (9.6) |
| | Arterial switch | 6 (8.2) |
| | Atrial septal defect repair | 2(2.7) |
| | LVOTO resection | 3 (4.1) |
| | valvular surgery | 6 (8.2) |
| | Aortic patch | 3 (4.1) |
| | others | 20 (27.4) |
| | | |
| 7-day mortality - no. (%) | | 0(0) |
| Post - OP ECMO (within 7 days) - no. (%) | | 0(0) |
| Length of hospital stay (days) - median (IQR) | | 14.0 (7.5; 22.0) |
| | Length of ICU stay (days) - median (IQR) | 4.0 (2.0; 7.0) |
| | | |

| Intraoperative variables - median (IQR) | | |
|---|---|---|
| | Length of surgery (min) | 230 (177.0; 278.5) |
| | Cardiopulmonary bypass time (min) | 96.2 (64.5; 142.0) |
| | Aortic clamp time (min) | 45.0 (0; 81.0) |
| | Volume therapy on cardiopulmonary bypass (mL/kg body weight | 27.7 (9.8; 48.5) |
| | Minimal body temperature, rectal (° Celsius) | 32.5 (31.9; 34.9) |
| | | |

| Post-operative variables: | | |
|---|---|---|
| | Intubation time (hours) - median (IQR) | 12.0 (10.0; 17.5) |
| | Length of catecholamine administration (hours) - median (IQR) | 25.0 (7.0; 78.0) |
| | Total fluid balance (mL/ kg body weight) (within 24 hours post-OP) - median (IQR) | -21.9 (-60.6; 12.0) |
| | Change of initial weight (%) - median (IQR) | 0.1 (-4.2; 4.1) |
| | Clinically relevant pleural effusion^{#} (within 9 days post-OP) no. (%) | 20 (27.4) |
| | Clinically relevant pericardial effusion^{#} (within 9 days post OP) - no. (%) | 2(2.7) |
| | Clinically relevant ascites (within 9 days post-OP) - no. (%) | 0 (0) |
| | Clinically relevant infection (within 9 days post-OP) - no. (%) | 8 (11.0) |
| | Clinically relevant sepsis (within 9 days post-OP) - no. (%) | 7 (9.6) |
| | | |
| Perioperative administration of corticosteroids - no. (%) | | 30 (41.1) |
| | Pre-operative dexamethasone | 18 (24.7) |
| | Post-operative hydrocortisone | 3 (4.1) |
| | Post-operative prednisone | 12 (16.4) |

### Bio-ADM levels and CPB

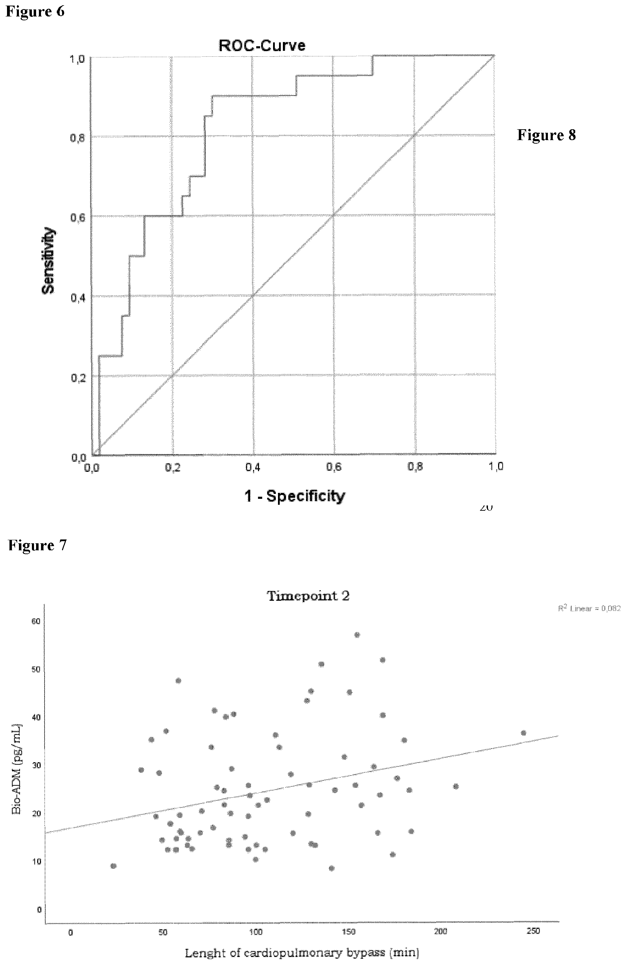

Bio-ADM levels were elevated in children after heart surgery on CPB (p<0.001). The median pre-operative bio-ADM (baseline) level was 12 pg/mL [IQR 12.0; 14.8] and the median intra-operative value was 21.3 pg/mL [IQR 14.3; 32.3]. Post-operative median bio-ADM concentrations of 33.5 pg/mL [22.5; 55.5] (TP 3), 38.8 pg/mL [25.5; 66.0] (TP 4) and 23.3 pg/mL [14.6; 34.4] (TP 5) were measured (Figure 5). The median of all maximum bio-ADM concentrations was 48.8 pg/mL [IQR 34.5; 69.6]. 41 (56.2%) of our studied patients reached a post-operative maximum bio-ADM level higher than 43 pg/mL (pre-defined cut-off) and 34 patients (46.6%) higher than 51.35 pg/mL (cut-off calculated with ROC curve, Figure 6).

### Correlation of bio-ADM with intra- and post-operative parameters

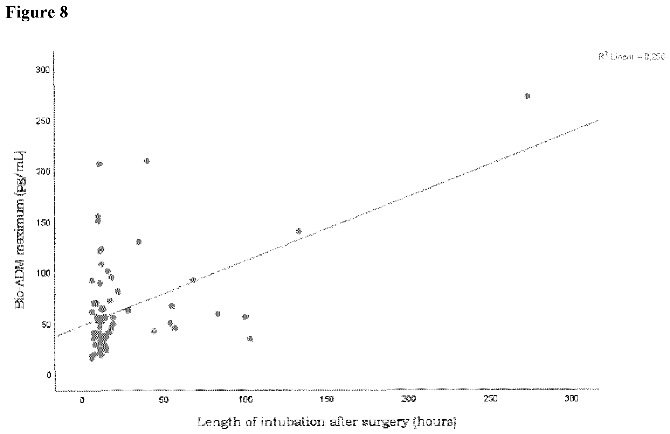

Median length of cardiopulmonary bypass was 96.2 (64.5 - 142.0) minutes. Intra-operative bio-ADM concentrations at the end of CPB (Timepoint 2) correlated with total bypass time (r=0.268; p<0.022; Figure 7). Additionally, maximum bio-ADM of all timepoints correlated with length of intubation (r=0.257; p<0.028; Figure 8. No significant relationship was found between bio-ADM levels and length of surgery as well as aortic clamp length. Children were admitted at the Intensive Care Unit (ICU) for 4 (2-7) days and were hospitalized for 14 (7.5-22) days. Maximum bio-ADM values were associated with length of hospital stay (r=0.584; p<0.001). No significant correlation was found between bio-ADM concentrations and length of ICU stay.

### Bio-ADM as a marker for endothelial dysfunction and capillary leak

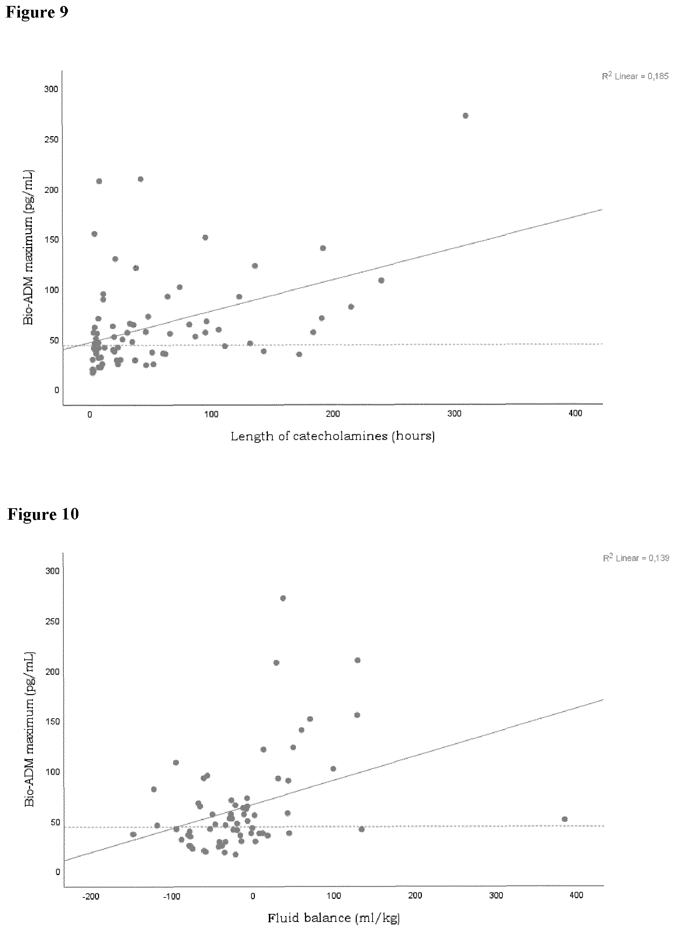

Bio-ADM concentrations show a relationship with different markers for endothelial dysfunction. In our studied patients, high bio-ADM levels were associated with long catecholamine requirement (r=0.415, p<0.001, Figure 9) and a higher amount of fluid balance (r=0.372, p<0.001, Figure 10). Median length of catecholamine demand was 25.0 (7.0; 78.0) hours and median fluid balance per kg body weight was -2.9 mL/ kg (-60.6; 12.0).

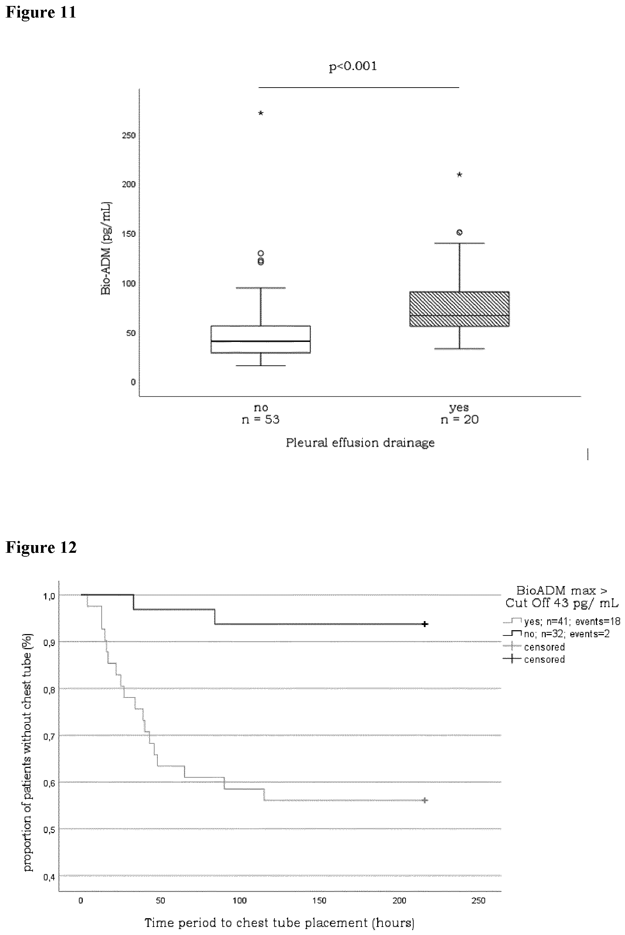

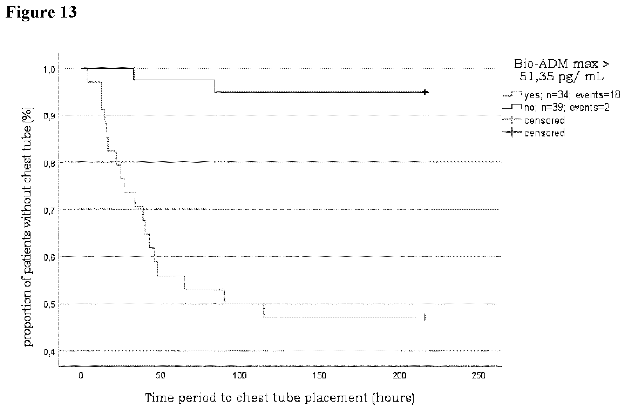

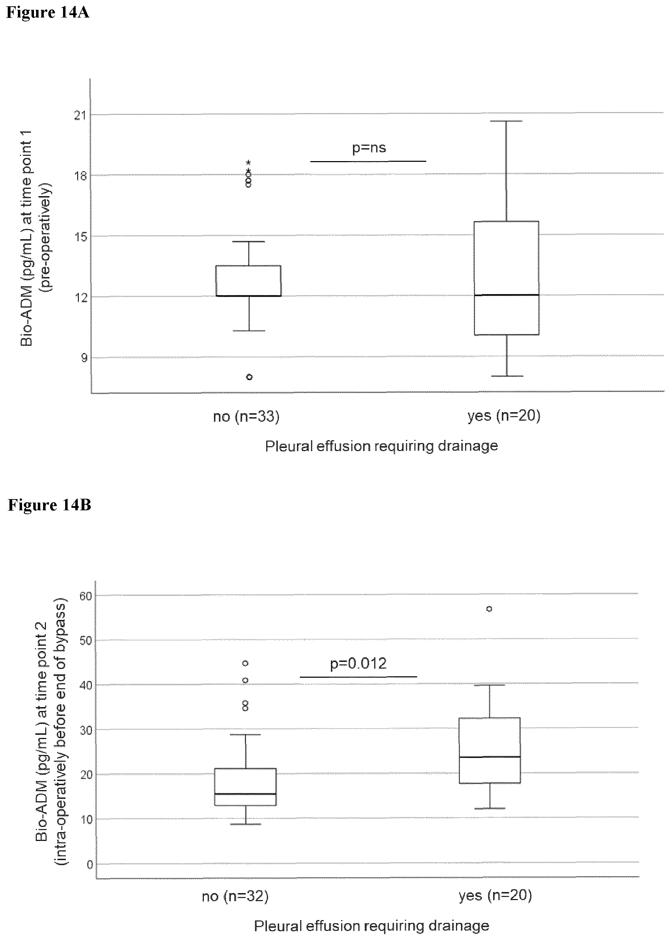

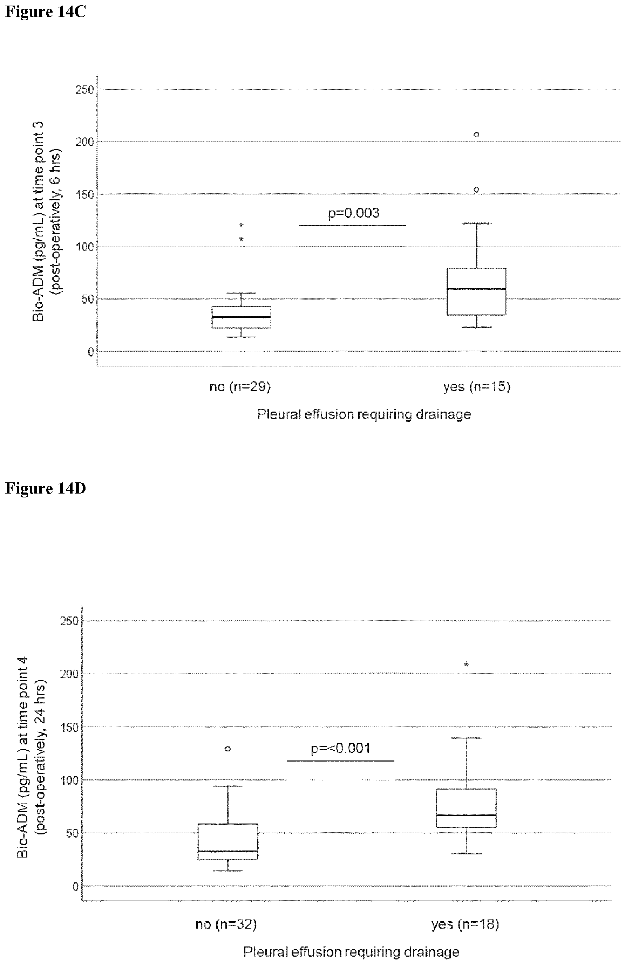

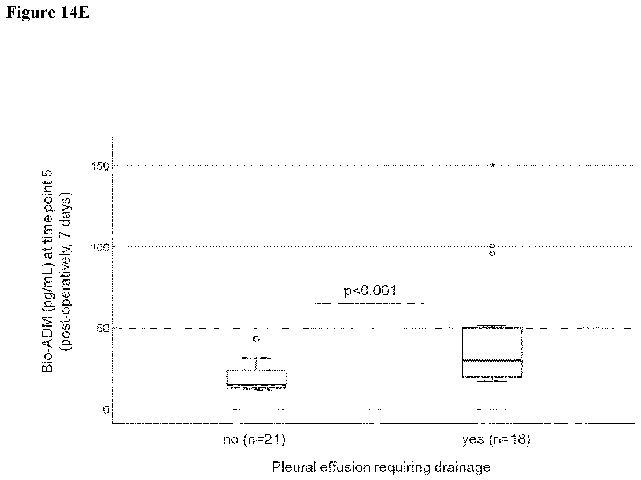

As a clinical marker for capillary leak, we examined the occurrence of post-operative pleural effusion that required invasive drainage in the observation period of nine days post-surgery. A pleural drainage was needed in 20 (27.4 %) of the studied cases. Bio-ADM levels strongly discriminated patients who required pleural effusion drainage after surgery from those who did not (p<0.001, Figure 11). Median (IQR) bio-ADM concentration in children without pleural drainage was 40.2 (28.2; 57.0) pg/mL compared to 66.4 (55.4; 90.9) pg/mL in the group with pleural drainage. With the predefined cutoff values of 43 pg/mL and 51.35 pg/mL Kaplan Meier curves were created that confirmed predictive potential of bio-ADM for the need of pleural effusion drainage (p<0.001; Figure 12, Figure 13). Patients required pleural effusion drainage after surgery exhibited significantly elevated bio-ADM concentrations in comparison to those who did not already at time point 2, that is intraoperatively (Figure 14). A significant elevation persisted through all post-operative timepoints assessed (Figure 14).

### Influence of post-operative infection and application of corticosteroids on bio-ADM value

### Subgroup Analysis

### Association of post-operative bio-ADM levels with infection and sepsis

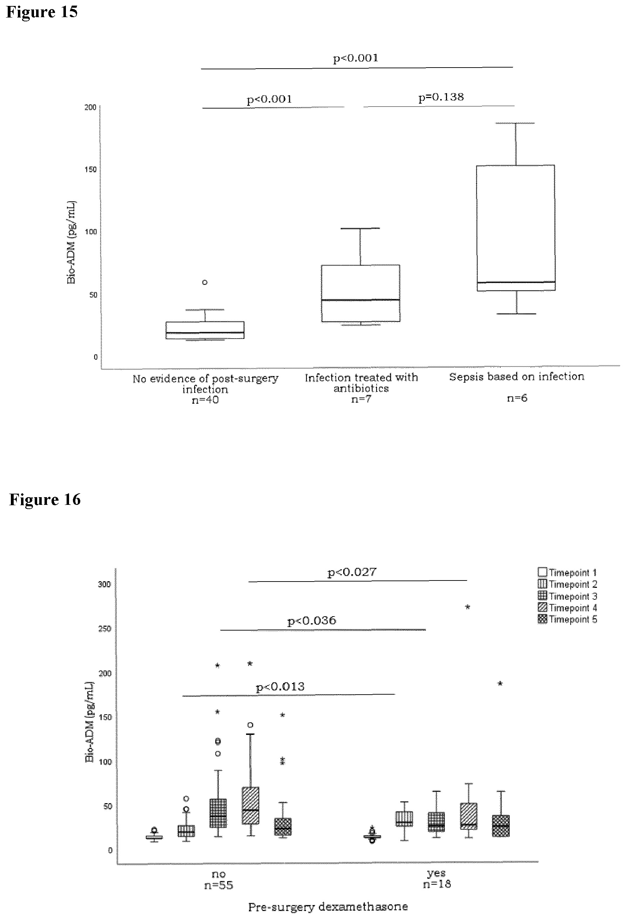

In accordance with prior studies, we examined the relationship between bio-ADM values and infection. Eight (11.0%) studied patients developed post-surgery infection that needed antibiotic treatment, seven (9.6%) patients fulfilled sepsis criteria. Figure 15 reveals that bio-ADM values are significantly higher in the infection and sepsis group compared to all other patients (p<0.001). Median (IQR) bio-ADM concentration in the infection group was 43.4.1 pg/mL (24.2; 96.0) and in the sepsis group 57.0 pg/mL (45.5; 158.7) compared to 18.1 pg/mL (13.1; 27.2) in the control group. No significant difference can be found in bio-ADM values between the infection and sepsis group.

### Effect of corticosteroids on post-operative bio-ADM levels

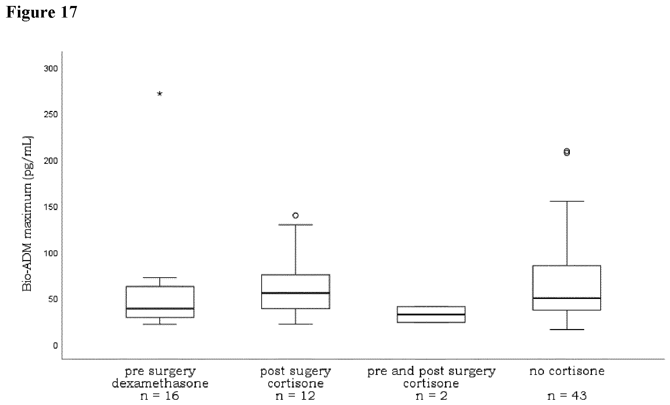

In a sub-analysis we examined the impact of cortisone treatment on bio-ADM concentrations. Whereas pre-surgery dexamethasone influences bio-ADM values at Timepoint 2, 3 and 4 significantly, post-surgery application of corticosteroids does not (Table ; Figure 16). Moreover, no significant difference can be found regarding the maximum bio-ADM concentration over all five timepoints and different timepoints of corticosteroid application (Figure 17).

**Table 5: Possible factors influencing bio-ADM values. Analysis of possible factors influencing bio-ADM values; Mann-Whitney-U test was performed for comparison of two variables, Kruskal-Wallis test for comparison of more than two variables.**

| | Bio-ADM TP1 median (IQR) | Bio-ADM TP2 median (IQR) | Bio-ADM TP3 median (IQR) | Bio-ADM TP4 median (IQR) | Bio-ADM TP5 median (IQR) | Bio-ADM max median (IQR) |
|---|---|---|---|---|---|---|
| Total (73) | 12.0 (12.0; 14.8) | 21.3 (14.3; 32.3) | 33.5 (22.5; 55.5) | 38.8 (25.5; 66.0) | 23.3 (14.6; 34.4) | 48.8 (34.5; 69.6) |
| | | | | | | |

| Gender | | | | | | |
|---|---|---|---|---|---|---|
| Male (41) | 12.0 (12.0; 15.2) | 24.2 (15.0; 34.0) | 36.2 (23.4; 57.7) | 49.6 (26.9; 69.3) | 23.5 (17.1; 33.9) | 54.5 (36.8; 70.6) |
| Female (32) | 12.0 (11.9; 14.1) | 20.3 (13.2; 27.8) | 32.8 (19.9; 50.4) | 32.7 (24.3; 64.3) | 21.2 (12.7; 44.1) | 40.8 (31.5; 64.3) |
| p-value (male vs. female) | 0.963 | 0.325 | 0.326 | 0.208 | 0.731 | 0.430 |
| | | | | | | |

| Age group | | | | | | |
|---|---|---|---|---|---|---|
| Infant (33) | 12.0 (12.0; 13.6) | 19.3 (13.5; 29.0) | 36.9 (24.0; 50.6) | 38.8 (27.3; 63.0) | 23.8 (14.4; 36.5) | 48.8 (35.8; 62.7) |
| Toddler + Preschool (26) | 12.0 (11.4; 15.0) | 21.8 (14.5; 29.7) | 39.2 (22.2; 74.5) | 60.7 (25.4; 80.8) | 20.4 (15.7; 34.9) | 62.1 (34.0; 105.5) |
| School Age (7) | 12.0 (9.8; 16.6) | 28.6 (23.2; 36.0) | 19.9 (12.0; 26.4) | 21.4 (19.5; 41.1) | 21.1 (12.5; 29.0) | 30.9 (23.2; 51.3) |
| Adolescent and Young (7) | 16.7 (9.1; 19.3) | 25.3 (15.3; 39.8) | 32.0 (19.5; 35.7) | 34.8 (15.1; 56.3) | 25.0 (12.0; 43.0) | 39.8 (36.4; 56.3) |
| p-value (age groups) | 0.761 | 0.453 | 0.43 | 0.159 | 0.889 | 0.123 |
| | | | | | | |

| Pre-surgery dexamethasone | | | | | | |
|---|---|---|---|---|---|---|
| Yes (18) | 12.0 (11.5; 14.6) | 28.3 (24.0; 40.9) | 24.6 (17.7; 40.2) | 25.2 (19.9; 55.0) | 23.5 (12.0; 46.8) | 38.1 (28.3; 61.4) |
| No (55) | 12.0 (12.0; 14.8) | 19.2 (14.0; 26.7) | 36.2 (23.9; 56.2) | 43.3 (27.3; 69.3) | 22.4 (15.0; 33.6) | 51.4 (36.5; 88.7) |
| p-value (yes vs. no) | 0.947 | 0.013 | 0.036 | 0.027 | 0.975 | 0.118 |
| | | | | | | |

| Post-surgery corticosteroids | | | | | | |
|---|---|---|---|---|---|---|
| Yes (14) | 12.0 (12.0; 15.1) | 23.2 (15.1; 31.7) | 31.7 (24.3; 47.4) | 48.8 (26.8; 74.8) | 17.8 (13.5; 38.8) | 51.7 (29.7; 65.2) |
| No (59) | 12.0 (12.0; 14.7) | 21.1 (14.3; 33.3) | 34.1 (21.7; 58.9) | 37.9 (24.1; 66.5) | 23.9 (14.6; 34.4) | 45.5 (34.5; 69.6) |
| p-values (yes vs. no) | 0.942 | 0.774 | 0.717 | 0.543 | 0.521 | 0.939 |
| | | | | | | |

| Infection/Sepsis | | | | | | |
|---|---|---|---|---|---|---|
| Infection (8) | 14.1 (11.9; 16.3) | 23.7 (20.6; 25.2) | 58.1 (50.6; 88.0) | 78.1 (65.1; 92.8) | 43.4 (24.2; 96.0) | 91.4 (67.8; 129.6) |
| Sepsis (7) | 12.0 (18.0; 20.6) | 25.4 (14.3; 50.5) | 60.5 (35.7; 121.0) | 78.9 (52.1; 190.5) | 57.0 (45.5; 158.7) | 120.1 (51.4; 150.2) |
| None (58) | 12.0 (12.0; 13.1) | 19.4 (13.7; 31.7) | 30.2 (21.3; 40.8) | 33.1 (23.9; 55.4) | 18.1 (13.1; 27.2) | 40.8 (30.7; 55.8) |
| p-value (infection status) | 0.025 | 0.331 | 0.005 | <0.001 | <0.001 | <0.001 |
| | | | | | | |

| Type of surgery | | | | | | |
|---|---|---|---|---|---|---|
| VSD (8) | 12.0 (12.0; 12.0) | 13.6 (12.0; 15.5) | 26.9 (19.5; 40.5) | 32.3 (26.2; 39.6) | 42.0 (25.7; xx) | 37.3 (28.6; 52.8) |
| PCPC (7) | 12.0 (12.0; 17.7) | 24.9 (14.3; 36.7) | 34.8 (25.7; 76.3) | 33.1 (23.9; 55.4) | 28.4 (20.5; 38.5) | 45.5 (34.8; 55.4) |
| TCPC (11) | 12.0 (8.3; 15.8) | 20.0 (14.0; 25.4) | 88.0 (64.4; 138.0) | 74.3 (64.4; 89.3) | 34.6 (19.6; 62.7) | 100.6 (69.6; 150.2) |
| cAVSD (7) | 12.0 (12.0; 14.2) | 23.2 (13.9; 40.9) | 38.8 (28.8; 47.4) | 38.8 (23.4; 60.1) | 14.2 (12.0; 35.3) | 40.9 (38.4; 48.8) |
| TGA (6) | 12.4 (12.0; 14.3) | 20.2 (18.1; 30.2) | 28.1 (22.6; 40.1) | 51.9 (30.9; 64.2) | 19.4 (14.1; 25.1) | 55.8 (44.0; 64.2) |
| ASD (2) | 11.0 (8.0; xx) | 18.7 (8.7; xx) | 17.7 (15.4; xx) | 17.8 (14.6; xx) | ------ | 22.0 (15.4; xx) |
| LVOTO resection (3) | 13.5 (12.0; xx) | 12.9 (12.9; xx) | 30.5 (19.1; xx) | 34.8 (17.6; xx) | 15.2 (12.0; xx) | 36.5 (19.1; xx) |
| Valvular surgery (6) | 12.8 (12.0; 18.8) | 37.4 (22.1; 50.7) | 36.1 (24.5; 58.2) | 43.9 (24.0; 68.7) | 22.4 (12.0; 37.3) | 59.0 (48.5; 68.7) |
| Aortic patch (3) | 9.8 (8.0; xx) | 24.3 (24.2; xx) | 17.0 (14.2; xx) | 19.1 (11.0; xx) | 27.8 (21.1; xx) | 24.3 (21.1; xx) |
| Others (20) | 12.0 (8.3; 16.2) | 24.1 (14.8; 30.7) | 31.0 (19.5; 56.1) | 34.9 (24.4; 81.6) | 18.1 (12.7; 23.7) | 38.3 (28.1; 67.8) |
| p-value (type of surgery) | 0.391 | 0.162 | 0.002 | 0.009 | 0.205 | <0.001 |

### Discussion

This study was a prospective single center study examining the impact of heart surgery on CPB on post-operative bio-ADM levels and the relationship between bio-ADM levels and clinical outcome.

### Bio-ADM levels and CPB

We found elevated bio-ADM values after surgery on CPB in the pediatric population. This is in accordance with prior studies that also describe increased ADM levels post-CPB (Nagata, N., et al. (1997) Anesth Analg 84; 1193-7, Takeuchi, M., et al. (2001) Acta Med Okayama 55; 245-52). However, the timepoint at which bio-ADM levels peak are controversial. Whereas Nagata et al. found highest ADM values at weaning from bypass (Nagata, N., et al. (1997) Anesth Analg 84; 1193-7), Takeuchi et al. measured the highest concentrations 60 minutes after end of CPB (Takeuchi, M., et al. (2001) Acta Med Okayama 55; 245-52). In this study, bio-ADM concentrations peaked at timepoint 4 (24h hours post-surgery). Additionally, we revealed significant correlation between length of cardiopulmonary bypass and maximum length of intubation with bio-ADM values, results that go in hand with findings by Bobillo-Perez et al. (Bobillo-Perez, S., et al. (2020) PloS one 15; e0236377) and suggest a relationship between bypass-dependent inflammatory processes and bio-ADM concentrations. However, these comparisons need to be interpreted with caution since the before mentioned prior reports did not measure the biologically active form bio-ADM that is suspected to be most closely related to the patient's clinical condition, but Adrenomedullin (ADM) that misses C-terminal amidation (Weber, J., et al. (2017) J Appl Lab Med 2; 222-33).

### Bio-ADM levels and clinical outcome

This study reveals a relationship between bio-ADM levels and the need for catecholamine support and volume therapy. Patients with high post-operative bio-ADM levels needed catecholamines over a longer period and a higher quantity of volume supplements. This is in accordance with other studies that showed correlation between ADM values and inotropic support in patients after surgery on CPB (Bobillo-Perez, S., et al. (2020) PloS one 15; e0236377) and in patients with sepsis or septic shock (Mebazaa, A., et al. (2018) Critical care 22; 354, Marino, R., et al. (2014) Critical care 18; R34, Kim, H, et al. (2019) Annals of laboratory medicine 39; 454-63). These findings may be explained by bio-ADM's vasodilatory properties (Geven, C., et al. (2018) Shock 50; 132-40, Geven, C., et al. (2018) Frontiers in immunology 9; 292) that may influence vasopressor demand and CPB dependent damage to the endothelium that triggers bio-ADM release.

As an additional marker for endothelial dysfunction, we examined bio-ADM levels in patients who required chest tube placement for pleural effusion and those who did not. Post-operative bio-ADM levels discriminated patients who developed pleural effusion from those who did not.

We illustrated the potential predictive properties of bio-ADM by applying the two cut-off values of 43 pg/mL (99^{th} percentile of the normal range) and 51.35 pg/mL (based on this cohort) on development of pleural effusion in the post-operative period. Patients with post-operative bio-ADM levels >43 pg/mL have a higher risk of pleural effusion compared to those with concentrations <43 pg/mL (Figure 12). The same accounts for the cut-off of 51.35 pg/mL (Figure 13). On the one hand pleural effusions can be a result of endothelial dysfunction and capillary leakage triggered by CPB dependent inflammatory processes (Seghaye, M.C. (2003) Cardiol Young 13; 228-39). On the other hand, hemodynamic processes in congenital heart diseases can lead to extravasation of fluid as well. Several studies in children with univentricular heart disease revealed a mean pulmonary artery pressure (mPAP) > 15 mmHg as risk factor for prolonged (>14 days) pleural effusion (Zou, M., et al. (2016) J Thorac Dis 8; 43-51, Rogers, L.S., et al. (2012) J Am Coll Cardiol 60; 1018-25). In this study patients after completed Fontan procedure (TCPC) had significant higher bio-ADM levels at timepoint 3 and 4 (Table ) and are especially represented in the group of patients who needed pleural effusion drainage (45% of the children with pleural effusion). Nevertheless, we could also reveal significant elevated bio-ADM values in patients with pleural effusion and heart surgery other than TCPC. This goes in hand with findings that relate prolonged pleural effusion to inflammatory processes and found correlation between bypass time and length (Francois, K., et al. (2009) Eur J Cardiothorac Surg 36; 57-62) as well as volume (Gupta, A., et al. (2004) J Thorac Cardiovasc Surg 127; 1664-9) of pleural effusion. Pleural effusions are a common complication after heart surgery and are associated with prolonged hospitalization (Vaynblat, M., et al. (1997) J Card Surg 12; 71-6). That is why a safe and reliable biomarker could be a useful tool to enable early initiation of necessary therapy and could be used as a medical target itself. One option to use bio-ADM for therapeutic interventions is Adrecizumab, a non-neutralizing antibody which binds bio-ADM at its N-terminus (Geven, C., et al. (2018) Frontiers in immunology 9; 292). Pre-clinical studies show that pre-treatment with Adrecizumab reduces catecholamine demand and attenuates kidney dysfunction in CLP-induced murine sepsis (Wagner, K., et al. (2013) Intensive care medicine experimental 1; 21). Additionally, it improved vascular barrier function in models of systemic inflammation and sepsis (Geven, C., et al. (2018) Shock 50; 648-54). The antibody's proposed mode of action is that it binds bio-ADM but is only marginally inhibiting it. Hence, administration of Adrecizumab leads to a net increase of bio-ADM in the circulation because the antibody's molecular weight makes crossing over the endothelial barrier impossible. Thus bio-ADM is kept in the intravascular compartment where it can exert its endothelium stabilizing functions while reduced bio-ADM concentrations in the interstitial space attenuate its vasodilatory effects (Geven, C., et al. (2018) Frontiers in immunology 9; 292, Geven, C., et al. (2018) Shock 50; 132-40, Geven, C. and Pickkers, P. (2018) Critical care 22; 159).

### Sub-analysis

Pre-treatment with corticosteroids is a common method to reduce post-CPB inflammation in cardiac surgery because they are known to reduce the amount of circulating pro-inflammatory mediators (Paparella, D., et al. (2002) Eur J Cardiothorac Surg 21; 232-44, Day, J.R. and Taylor, K.M. (2005) Int J Surg 3; 129-40). However, there are studies that argue the beneficial effects on clinical outcome and could show that administration of cortisone did not reduce post-surgery complications and mortality (Lomivorotov, V., et al. (2020) JAMA 323; 2485-92, Kristeller, J.L., et al. (2014) Hosp Pharm 49; 232-6). In this study bio-ADM values at timepoint 2,3 and 4 are significantly lower in the group of patients pre-treated with dexamethasone before surgery compared to the group that was not. Application of corticosteroids in the post-operative period did not influence bio-ADM concentrations significantly. A reason for this finding could be that during surgery inflammatory processes already started and as a consequence post-surgery application of corticosteroids was not as effective anymore. No impact of pre- or post-surgery corticosteroid administration was found on overall maximum bio-ADM concentrations.

Bio-ADM is well examined in the context of sepsis and septic shock. In accordance with prior studies we revealed elevated bio-ADM levels in patients with infection that required antibiotic treatment and in those that additionally fulfilled SIRS criteria (Marino, R., et al. (2014) Critical care 18; R34, Kim, H., et al. (2019) Annals of laboratory medicine 39; 454-63, Mebazaa, A., et al. (2018) Critical care 22; 354, Lundberg, O.H.M., et al. (2020) Critical care 24; 636). The interpretation of these finding is limited by the fact that this study does not include sepsis patients at the first place but children who developed post-surgery infection.

Limitations included that in the present study only patients who had surgery including CPB were enrolled. Future studies should consider patients who need heart surgery without CPB in order to find out whether the trauma of surgery itself or the use of CPB has the main impact on bio-ADM values. Additionally, blood samples could only be taken within routine blood draws out of ethical reasons. Hence blood sampling timepoints could not always be kept which might has influenced bio-ADM measurements.

### Conclusion

In this study we revealed elevated bio-ADM levels in children after heart surgery on CPB and showed a strong relationship between bio-ADM concentrations and clinical outcome. Since capillary leak syndrome in children is associated with increased morbidity bio-ADM should be considered as a potential biomarker and medical target for therapy.

### Sequences

SEQ ID No. 1 (proADM): 164 amino acids (22 - 185 of preproADM) 
ARLDVASEF RKKWNKWALS RGKRELRMSS SYPTGLADVK AGPAQTLIRP
QDMKGASRSP EDSSPDAARI RVKRYRQSMN NFQGLRSFGC RFGTCTVQKL
AHQIYQFTDK DKDNVAPRSK ISPQGYGRRR RRSLPEAGPG RTLVSSKPQA
HGAPAPPSGS APHFL
SEQ ID NO. 2 : N-terminal part (amino acids 1-21) of of mature human ADM
   YRQSMNNFQGLRSFGCRFGTC
SEQ ID No. 3 (Proadrenomedullin N-20 terminal peptide, PAMP): amino acids 22 - 41 of preproADM
   ARLDVASEF RKKWNKWALS R
SEQ ID No. 4 (Midregional proAdrenomedullin, MR-proADM): amino acids 45 - 92 of preproADM
   ELRMSS SYPTGLADVK AGPAQTLIRP QDMKGASRSP EDSSPDAARI RV
SEQ ID No. 5 (mature Adrenomedullin (mature ADM); amidated ADM; bio-ADM): amino acids 95 - 146 -CONH₂ 
YRQSMN NFQGLRSFGC RFGTCTVQKL AHQIYQFTDK DKDNVAPRSK ISPQGY -
CONH2
SEQ ID No. 6 (Adrenomedullin 1-52-Gly (ADM 1-52-Gly)): amino acids 95 - 147 of preproADM
   YRQSMN NFQGLRSFGC RFGTCTVQKL AHQIYQFTDK DKDNVAPRSK ISPQGYG
SEQ ID No. 7 (C-terminal proAdrenomedullin, CT-proADM): amino acids 148 - 185 of preproADM
   RRR RRSLPEAGPG RTLVSSKPQA HGAPAPPSGS APHFL
SEQ ID NO: 8:
   GYTFSRYW
SEQ ID NO: 9:
   ILPGSGST
SEQ ID NO: 10:
   TEGYEYDGFDY
SEQ ID NO: 11
   QSIVYSNGNTY
   (not part of the sequence protocol):
   RVS
CDR3: SEQ ID NO: 12
   FQGSHIPYT.
SEQ ID No.: 13 (AM-VH-C) 
QVQLQQSGAELMKPGASVKISCKATGYTFSRYWIEWVKQRPGHGLEWIGEILPGSGS
TNYNEKFKGKATITADTSSNTAYMQLSSLTSEDSAVYYCTEGYEYDGFDYWGQGTT
LTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKHHHHHH
SEQ ID No.: 14 (AM-VH1) 
QVQLVQSGAEVKKPGSSVKVSCKASGYTFSRYWISWVRQAPGQGLEWMGRILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH
SEQ ID No.: 15 (AM-VH2-E40) 
QVQLVQSGAEVKKPGSSVKVSCKASGYTFSRYWIEWVRQAPGQGLEWMGRILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH
SEQ ID No.: 16 (AM-VH3-T26-E55) 
QVQLVQSGAEVKKPGSSVKVSCKATGYTFSRYWISWVRQAPGQGLEWMGEILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH
SEQ ID No.: 17 (AM-VH4-T26-E40-E55) 
QVQLVQSGAEVKKPGSSVKVSCKATGYTFSRYWIEWVRQAPGQGLEWMGEILPGSG
STNYAQKFQGRVTITADESTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKHHHHHH
SEQ ID No.: 18 (AM-VL-C) 
DVLLSQTPLSLPVSLGDQATISCRSSQSIVYSNGNTYLEWYLQKPGQSPKLLIYRVSNR
FSGVPDRFSGSGSGTDFTLKISRVEAEDLGVYYCFQGSHIPYTFGGGTKLEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC
SEQ ID No.: 19 (AM-VL1) 
DVVMTQSPLSLPVTLGQPASISCRSSQSIVYSNGNTYLNWFQQRPGQSPRRLIYRVSN
RDSGVPDRFSGSGSGTDFTLKISRVEAEDVGVYYCFQGSHIPYTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID No.: 20 (AM-VL2-E40) 
DVVMTQSPLSLPVTLGQPASISCRSSQSIVYSNGNTYLEWFQQRPGQSPRRLIYRVSN
RDSGVPDRFSGSGSGTDFTLKISRVEAEDVGVYYCFQGSHIPYTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 22 (heavy chain, HAM8101) 
QVQLVQSGAEVKKPGSSVKVSCKASGYTFSRYWIEWVRQAPGQGLEWIGEILPGSGS
TNYNQKFQGRVTITADTSTSTAYMELSSLRSEDTAVYYCTEGYEYDGFDYWGQGTT
VTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
SEQ ID NO: 23 (light chain, HAM8101) 
DVVLTQSPLSLPVTLGQPASISCRSSQSIVYSNGNTYLEWYLQRPGQSPRLLIYRVSNR
FSGVPDRFSGSGSGTDFTLKISRVEAEDVGVYYCFQGSHIPYTFGGGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID No.: 24 (human ADM 21-32)
   CTVQKLAHQIYQ
SEQ ID No.: 25 (human ADM C-42-52)
   CAPRSKISPQGY-CONH₂
SEQ ID No.: 26 (murine ADM 1-19)
   YRQSMNQGSRSNGCRFGTC
SEQ ID No.: 27 (murine ADM 19-31)
   CTFQKLAHQIYQ
SEQ ID No.: 28 (murine ADM C-40-50)
   CAPRNKISPQGY-CONH₂

### References

1. Day JR, Taylor KM, (2005) The systemic inflammatory response syndrome and cardiopulmonary bypass. Int J Surg 3: 129-40
2. Elgebaly SA, Houser SL, el Kerm AF, Doyle K, Gillies C, Dalecki K, (1994) Evidence of cardiac inflammation after open heart operations. Ann Thorac Surg 57: 391-6
3. Cremer J, Martin M, Redl H, Bahrami S, Abraham C, Graeter T, Haverich A, Schlag G, Borst HG, (1996) Systemic inflammatory response syndrome after cardiac operations. Ann Thorac Surg 61: 1714-20
4. Kirklin JK, Blackstone EH, Kirklin JW, (1987) Cardiopulmonary bypass: studies on its damaging effects. Blood Purif 5: 168-78
5. Levy JH, Kelly AB, (1993) Inflammation and cardiopulmonary bypass. Can J Anaesth 40: 1009-15
6. Prasad K, Kalra J, Bharadwaj B, Chaudhary AK, (1992) Increased oxygen free radical activity in patients on cardiopulmonary bypass undergoing aortocoronary bypass surgery. American heart journal 123: 37-45
7. Seghaye MC, Grabitz RG, Duchateau J, Busse S, Dabritz S, Koch D, Alzen G, Hornchen H, Messmer BJ, Von Bernuth G, (1996) Inflammatory reaction and capillary leak syndrome related to cardiopulmonary bypass in neonates undergoing cardiac operations. J Thorac Cardiovasc Surg 112: 687-97
8. Tarnok A, Hambsch J, Emmrich F, Sack U, van Son J, Bellinghausen W, Borte M, Schneider P, (1999) Complement activation, cytokines, and adhesion molecules in children undergoing cardiac surgery with or without cardiopulmonary bypass. Pediatr Cardiol 20: 113-25
9. Teelucksingh S, Padfield PL, Edwards CR, (1990) Systemic capillary leak syndrome. Q J Med 75: 515-24
10. Seghaye MC, (2003) The clinical implications of the systemic inflammatory reaction related to cardiac operations in children. Cardiol Young 13: 228-39
11. Kubicki R, Grohmann J, Siepe M, Benk C, Humburger F, Rensing-Ehl A, Stiller B, (2013) Early prediction of capillary leak syndrome in infants after cardiopulmonary bypass. Eur J Cardiothorac Surg 44: 275-81
12. Zhu XB, Wang YB, Hao FZ, Zhang ZH, Chen SJ, (2006) [Plasma levels of adrenomedullin in children with congenital heart disease]. Zhongguo Dang Dai Er Ke Za Zhi 8: 90-2
13. Abella R, Satriano A, Frigiola A, Varrica A, Gavilanes AD, Zimmermann LJ, Vles HJ, Florio P, Calevo MG, Gazzolo D, (2012) Adrenomedullin alterations related to cardiopulmonary bypass in infants with low cardiac output syndrome. J Matern Fetal Neonatal Med 25: 2756-61
14. Wu AH, Hale K, (2015) Predicting mortality after elective open-heart surgery using midregional-proadrenomedullin: is it time to scalp Acute Physiology and Chronic Health Evaluation IV? Critical care medicine 43: 494-5
15. Takeuchi M, Morita K, Iwasaki T, Toda Y, Oe K, Taga N, Hirakawa M, (2001) Significance of adrenomedullin under cardiopulmonary bypass in children during surgery for congenital heart disease. Acta Med Okayama 55: 245-52
16. Perez-Navero JL, de la Torre-Aguilar MJ, Ibarra de la Rosa I, Gil-Campos M, Gomez-Guzman E, Merino-Cejas C, Munoz-Villanueva MC, Llorente-Cantarero FJ, (2017) Cardiac Biomarkers of Low Cardiac Output Syndrome in the Postoperative Period After Congenital Heart Disease Surgery in Children. Rev Esp Cardiol (Engl Ed) 70: 267-74
17. Bobillo-Perez S, Girona-Alarcon M, Corniero P, Sole-Ribalta A, Balaguer M, Esteban E, Valls A, Jordan I, Cambra FJ, (2020) Pro-atrial natriuretic peptide and pro-adrenomedullin before cardiac surgery in children. Can we predict the future? PloS one 15: e0236377
18. Bobillo-Perez S, Girona-Alarcon M, Canizo D, Camprubi-Camprubi M, Rodriguez-Fanjul J, Balaguer M, Benito S, Valls A, Cambra FJ, Jordan I, (2021) Mid-regional pro-adrenomedullin for diagnosing evolution after cardiac surgery in newborns: the PRONEW study. Eur J Pediatr
19. Goldstein B, Giroir B, Randolph A, International Consensus Conference on Pediatric S, (2005) International pediatric sepsis consensus conference: definitions for sepsis and organ dysfunction in pediatrics. Pediatr Crit Care Med 6: 2-8
20. Weber J, Sachse J, Bergmann S, Sparwasser A, Struck J, Bergmann A, (2017) Sandwich Immunoassay for Bioactive Plasma Adrenomedullin. J Appl Lab Med 2: 222-33
21. Marino R, Struck J, Maisel AS, Magrini L, Bergmann A, Di Somma S, (2014) Plasma adrenomedullin is associated with short-term mortality and vasopressor requirement in patients admitted with sepsis. Critical care 18: R34
22. Nagata N, Kitamura K, Kato J, Naruo H, Eto T, Takasaki M, (1997) The effect of hypothermic cardiopulmonary bypass on plasma adrenomedullin in adult cardiac surgical patients. Anesth Analg 84: 1193-7
23. Mebazaa A, Geven C, Hollinger A, Wittebole X, Chousterman BG, Blet A, Gayat E, Hartmann O, Scigalla P, Struck J, Bergmann A, Antonelli M, Beishuizen A, Constantin JM, Damoisel C, Deye N, Di Somma S, Dugernier T, Francois B, Gaudry S, Huberlant V, Lascarrou JB, Marx G, Mercier E, Oueslati H, Pickkers P, Sonneville R, Legrand M, Laterre PF, Adren OSSsi, (2018) Circulating adrenomedullin estimates survival and reversibility of organ failure in sepsis: the prospective observational multinational Adrenomedullin and Outcome in Sepsis and Septic Shock-1 (AdrenOSS-1) study. Critical care 22: 354
24. Kim H, Hur M, Struck J, Bergmann A, Di Somma S, (2019) Circulating Biologically Active Adrenomedullin Predicts Organ Failure and Mortality in Sepsis. Annals of laboratory medicine 39: 454-63
25. Geven C, Bergmann A, Kox M, Pickkers P, (2018) Vascular Effects of Adrenomedullin and the Anti-Adrenomedullin Antibody Adrecizumab in Sepsis. Shock 50: 132-40
26. Geven C, Kox M, Pickkers P, (2018) Adrenomedullin and Adrenomedullin-Targeted Therapy As Treatment Strategies Relevant for Sepsis. Frontiers in immunology 9: 292
27. Zou M, Wang Y, Cui H, Ma L, Yang S, Xia Y, Chen W, Chen X, (2016) Outcomes of total cavopulmonary connection for single ventricle palliation. J Thorac Dis 8: 43-51
28. Rogers LS, Glatz AC, Ravishankar C, Spray TL, Nicolson SC, Rychik J, Rush CH, Gaynor JW, Goldberg DJ, (2012) 18 years of the Fontan operation at a single institution: results from 771 consecutive patients. J Am Coll Cardiol 60: 1018-25
29. Francois K, Bove T, De Groote K, Panzer J, Vandekerckhove K, Suys B, De Wolf D, Van Nooten G, (2009) Pleural effusions, water balance mediators and the influence of lisinopril after completion Fontan procedures. Eur J Cardiothorac Surg 36: 57-62
30. Gupta A, Daggett C, Behera S, Ferraro M, Wells W, Starnes V, (2004) Risk factors for persistent pleural effusions after the extracardiac Fontan procedure. J Thorac Cardiovasc Surg 127: 1664-9
31. Vaynblat M, Chiavarelli M, Anderson JE, Rao S, Nudel DB, Cunningham JN, Jr., (1997) Pleural drainage after repair of tetralogy of Fallot. J Card Surg 12: 71-6
32. Wagner K, Wachter U, Vogt JA, Scheuerle A, McCook O, Weber S, Groger M, Stahl B, Georgieff M, Moller P, Bergmann A, Hein F, Calzia E, Radermacher P, Wagner F, (2013) Adrenomedullin binding improves catecholamine responsiveness and kidney function in resuscitated murine septic shock. Intensive care medicine experimental 1: 21
33. Geven C, Peters E, Schroedter M, Struck J, Bergmann A, McCook O, Radermacher P, Kox M, Pickkers P, (2018) Effects of the Humanized Anti-Adrenomedullin Antibody Adrecizumab (HAM8101) on Vascular Barrier Function and Survival in Rodent Models of Systemic Inflammation and Sepsis. Shock 50: 648-54
34. Geven C, Pickkers P, (2018) The mechanism of action of the adrenomedullin-binding antibody adrecizumab. Critical care 22: 159
35. Paparella D, Yau TM, Young E, (2002) Cardiopulmonary bypass induced inflammation: pathophysiology and treatment. An update. Eur J Cardiothorac Surg 21: 232-44
36. Lomivorotov V, Kornilov I, Boboshko V, Shmyrev V, Bondarenko I, Soynov I, Voytov A, Polyanskih S, Strunin O, Bogachev-Prokophiev A, Landoni G, Nigro Neto C, Oliveira Nicolau G, Saurith Izquierdo L, Nogueira Nascimento V, Wen Z, Renjie H, Haibo Z, Bazylev V, Evdokimov M, Sulejmanov S, Chernogrivov A, Ponomarev D, (2020) Effect of Intraoperative Dexamethasone on Major Complications and Mortality Among Infants Undergoing Cardiac Surgery: The DECISION Randomized Clinical Trial. JAMA 323: 2485-92
37. Kristeller JL, Jankowski A, Reinaker T, (2014) Role of corticosteroids during cardiopulmonary bypass. Hosp Pharm 49: 232-6
38. Lundberg OHM, Lengquist M, Spangfors M, Annborn M, Bergmann D, Schulte J, Levin H, Melander O, Frigyesi A, Friberg H, (2020) Circulating bioactive adrenomedullin as a marker of sepsis, septic shock and critical illness. Critical care 24: 636

## Claims

1. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of Pro-Adrenomedullin (pro-ADM) or fragments thereof above a predetermined threshold in a sample of bodily fluid of said patient,
wherein said Pro-Adrenomedullin or fragment is selected from the group comprising Pro-Adrenomedullin according to SEQ ID No. 1 or PAMP according to SEQ ID No.: 3 or MR-proADM according to SEQ ID No.: 4 or mature ADM-NH2 (bio-ADM) according to SEQ ID No.: 5 or ADM-Gly according to SEQ ID No.: 6 or CT-proADM according to SEQ ID No.: 7.

2. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to claim 1, wherein said antibody or antibody fragment binds to the N-terminal part (aa 1-21) of ADM having the SEQ ID No. 2 (YRQSMNNFQGLRSFGCRFGTC) and wherein said antibody or antibody fragment is administered to a patient having a level of pro-Adrenomedullin or fragments thereof or fragments thereof above a predetermined threshold in a sample of bodily fluid of said patient.

3. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, according to claim 1 and 2, and wherein said antibody or antibody fragment is administered to a patient with a level of pro-Adrenomedullin or fragments thereof above a predetermined threshold which is the 95th percentile of the normal range of pro-Adrenomedullin or fragments thereof of a healthy subject, more preferred which is the 96th percentile of the normal range of bio-ADM of a healthy subject, even more preferred which is the 97th percentile of the normal range of of a healthy subject, wherein said sample of bodily fluid is selected from the group comprising whole blood, plasma, and serum, in particular blood plasma.

4. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, according to claim 1 to 3, wherein said antibody or antibody fragment is administered to a patient with a level of pro-Adrenomedullin or fragments thereof above a predetermined threshold, which is for the amount of bio-ADM in a sample of bodily fluid ≥ 43 pg/ml, wherein said sample of bodily fluid is selected from the group comprising whole blood, plasma, and serum, in particular blood plasma.

5. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to claim 1 to 4 and wherein said antibody or antibody fragment is administered to a patient having a level of pro-Adrenomedullin or fragments thereof, wherein such level is determined from a sample of bodily fluid taken pre-, intra- or postoperatively.

6. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to claim 1 to 5 and wherein said antibody or antibody fragment is administered to a patient having a level of pro-Adrenomedullin or fragments thereof, wherein the level of pro-Adrenomedullin or fragments thereof is determined in a bodily fluid obtained from said patient is measured with an immunoassay.

7. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to any of claims 1 to 6, wherein said antibody or antibody fragment may be administered in a dose of at least 0.5 mg / Kg body weight, particularly at least 1.0 mg/kg body weight, more particularly, from 1.0 to 20.0 mg/kg body weight, e.g., from 2.0 to 10 mg/kg body weight, from 2.0 to 8.0 mg/kg body weight, or from 2.0 to 5.0 mg/kg body weight.

8. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to any of claims 1 to 7, wherein said antibody or antibody fragment is a human monoclonal antibody or a fragment that binds to the N-terminal region (aa 1-21) of ADM (SEQ ID No. 2) or an antibody fragment thereof wherein the heavy chain comprises the sequences:
CDR1: SEQ ID NO: 8
GYTFSRYW
CDR2: SEQ ID NO: 9
ILPGSGST
CDR3: SEQ ID NO: 10
TEGYEYDGFDY
and wherein the light chain comprises the sequences:
CDR1: SEQ ID NO: 11
QSIVYSNGNTY
CDR2:
RVS
CDR3: SEQ ID NO: 12
FQGSHIPYT.

9. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass, according to any of claims 1 to 8, wherein said antibody or fragment comprises the following sequence as a heavy chain: 
SEQ ID NO: 22

QVQLVQSGAEVKKPGSSVKVSCKASGYTFSRYWIEWVRQAPGQGLEWIGEILPGSGSTN
YNQKFQGRVTITADTSTSTAYMELSSLRSEDTAVYYCTEGYEYDGFDYWGQGTTVTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
or a sequence that is > 95% identical to it,
and comprises the following sequence as a light chain: SEQ ID NO: 23 
DVVLTQSPLSLPVTLGQPASISCRSSQSIVYSNGNTYLEWYLQRPGQSPRLLIYRVSNRFSG
VPDRFSGSGSGTDFTLKISRVEAEDVGVYYCFQGSHIPYTFGGGTKLEIKRTVAAPSVFIFP
PSDEQLKSGTASVVCLLNNFYPREAKVQWKVDNALQSGNSQESVTEQDSKDSTYSLSSTL
TLSKADYEKHKVYACEVTHQGLSSPVTKSFNRGEC
or a sequence that is > 95% identical to it.

10. Anti-adrenomedullin (ADM) antibody or antibody fragment for the prevention or treatment of a pleural effusion in a pediatric patient with congenital heart disease that is undergoing or underwent cardiac surgery with cardio-pulmonary bypass according to any of claims 1 to 9, wherein said antibody or antibody fragments are administered orally, intravenously, subcutaneously, intraarterially, intramuscularly, intracardially, intraspinally, intrathoracically, intraperitoneal, intraventricular, sublingually, transdermal, and/or via inhalation.
